# EUROPEAN PATENT SPECIFICATION

(11) **EP 3 481 433 B1**
(45) Date of publication and mention of the grant of the patent: **03.07.2024**
(21) Application number: 17824815.9
(22) Date of filing: 05.07.2017
(51) Int. Cl.: A61K 48/00, C07K 14/005, C12N 7/00, C12N 15/86

(54) **AAV2-MEDIATED GENE DELIVERY OF SFASL AS A NEUROPROTECTIVE THERAPY IN GLAUCOMA**
AAV2-VERMITTELTE GENABGABE VON SFASL ALS NEUROPROTEKTIVE THERAPIE BEI GLAUKOM
ADMINISTRATION DE GÈNE À MÉDIATION PAR AAV2 DE SFASL COMME THÉRAPIE NEUROPROTECTRICE DANS LE GLAUCOME

(30) Priority: 05.07.2016 US 201662358541 P; 26.05.2017 US 201762511629 P
(43) Date of publication of application: 15.05.2019
(73) Proprietor: University of Massachusetts, Boston, MA 02108 (US); Massachusetts Eye & Ear Infirmary, Boston, MA 02114 (US)
(72) Inventor: MARSHAK-ROTHSTEIN, Ann, Newton, MA 02459 (US); GREGORY-KSANDER, Meredith, Boston, MA 02114 (US); KSANDER, Bruce, Boston, MA 02114 (US)
(74) Representative: Mewburn Ellis LLP
(86) International application number: PCT/US2017/040735
(87) International publication number: WO 2018/009553

(56) References cited:
- WO-A1-2015/044462
- US-A1- 2002 192 823
- US-A1- 2009 239 240
- US-A1- 2011 217 760
- US-A1- 2013 209 395
- US-A1- 2015 044 237
- US-A1- 2016 017 005
- KRISHNAN ANITHA ET AL: "Gene therapy treatment with AAV-soluble Fas ligand protects retinal ganglion cells during development of Glaucoma", INVESTIGATIVE OPHTHALMOLOGY & VISUAL SCIENCE, ASSOCIATION FOR RESEARCH IN VISION AND OPHTHALMOLOGY, US , vol. 56, no. 7 31 May 2015 (2015-05-31), page 2594, XP009518104, ISSN: 0146-0404 Retrieved from the Internet: URL:https://iovs.arvojournals.org/article. aspx?articleid=2332375&resultClick=1
- MEREDITH S. GREGORY ET AL: "Opposing Roles for Membrane Bound and Soluble Fas Ligand in Glaucoma-Associated Retinal Ganglion Cell Death", PLOS ONE, vol. 6, no. 3, 29 March 2011 (2011-03-29), page e17659, XP055657749, DOI: 10.1371/journal.pone.0017659
- H MATSUMOTO ET AL: "Membrane-bound and soluble Fas ligands have opposite functions in photoreceptor cell death following separation from the retinal pigment epithelium", CELL DEATH & DISEASE, vol. 6, no. 11, 1 November 2015 (2015-11-01), pages e1986-e1986, XP055657750, DOI: 10.1038/cddis.2015.334
- Qiuhong Li ET AL: "Intraocular route of AAV2 vector administration defines humoral immune response and therapeutic potential", Molecular vision, 24 September 2008 (2008-09-24), pages 1760-1769, XP055657882, United States Retrieved from the Internet: URL:https://www.ncbi.nlm.nih.gov/pmc/artic les/PMC2559816/pdf/mv-v14-1760.pdf
- SURACE ET AL: "Versatility of AAV vectors for retinal gene transfer", VISION RESEARCH, PERGAMON PRESS, OXFORD, GB, vol. 48, no. 3, 17 October 2007 (2007-10-17), pages 353-359, XP022452229, ISSN: 0042-6989, DOI: 10.1016/J.VISRES.2007.07.027
- ALMASIEH MOHAMMADALI ET AL: "The molecular basis of retinal ganglion cell death in glaucoma", PROGRESS IN RETINAL AND EYE RESEARCH, vol. 31, no. 2, 4 December 2011 (2011-12-04), pages 152-181, XP028896188, ISSN: 1350-9462, DOI: 10.1016/J.PRETEYERES.2011.11.002
- ANITHA KRISHNAN ET AL: "Overexpression of Soluble Fas Ligand following Adeno-Associated Virus Gene Therapy Prevents Retinal Ganglion Cell Death in Chronic and Acute Murine Models of Glaucoma", THE JOURNAL OF IMMUNOLOGY, vol. 197, no. 12, 14 November 2016 (2016-11-14), pages 4626-4638, XP055657617, ISSN: 0022-1767, DOI: 10.4049/jimmunol.1601488

## Description

### RELATED APPLICATIONS

This application claims the benefit under 35 U.S.C. 119(e) of U.S. provisional application serial number 62/511,629, filed May 26, 2017, and entitled "AAV2-MEDIATED GENE DELIVERY OF SFASL AS A NEUROPROTECTIVE THERAPY IN GLAUCOMA", and U.S. provisional application serial number 62/358,541, filed July 5, 2016, entitled "AAV2-MEDIATED GENE DELIVERY OF SFASL AS A NEUROPROTECTIVE THERAPY IN GLAUCOMA".

### BACKGROUND

Glaucoma, a leading cause of blindness worldwide, is a complex multifactorial disease characterized by the progressive loss of retinal ganglion cells (RGCs). Elevated intraocular pressure (IOP) is a well-recognized risk factor for the development of glaucoma, and remains the only modifiable disease-associated parameter. However, reduction of IOP alone does not prevent loss of RGCs in all patients and RGC destruction can continue even after IOP has been successfully lowered. Furthermore, a high incidence of glaucoma with loss of RGCs occurs in patients having normal IOP.

Krishnan et al. (Investigative Ophthalmology & Visual Science June 2015, Vol.56, 2594) disclosed that mFasL prevents RGC loss in mouse models of IOP-induced glaucoma.

### SUMMARY

The invention is defined in the claims.

Aspects of the disclosure relate to compositions and methods for preventing optic nerve degeneration. In some embodiments, compositions and methods provided herein are useful for treating glaucoma. In some embodiments, the present disclosure relates to the discovery that Fas ligand (FasL) mediates apoptosis and inflammation that results in tissue damage, *e.g.*, in glaucoma. In some embodiments, the present disclosure relates to the discovery that rAAV-mediated delivery of soluble Fas ligand (sFasL) prevents death of retinal ganglion cells (RGCs) and axons in a mouse model of glaucoma.

Accordingly, one aspect of the present disclosure provides compositions for use in methods for treating glaucoma in a subject. In some embodiments, the methods involve administering to a subject in need thereof an effective amount of recombinant adeno-associated virus (rAAV), in which the rAAV comprises (i) capsid protein and (ii) a nucleic acid engineered to express sFasL or a fragment thereof. In some examples, the amount of sFasL or a fragment thereof is effective in reducing glaucoma disease progression, lowering intraocular pressure in the subject, inactivating retinal glial cells, inhibiting TNFα activity, reducing retinal ganglion cell (RGC) death, and/or reducing axonal degeneration. In some embodiments, novel compositions and methods are provided for preventing optic nerve degeneration and/or for treating glaucoma.

In another aspect, the present disclosure provides compositions for use in methods for treating a Fas ligand-dependent inflammatory condition. In some embodiments, the methods involve administering to a subject in need thereof an effective amount of recombinant adeno-associated virus (rAAV), wherein the rAAV comprises (i) capsid protein and (ii) a nucleic acid engineered to express sFasL or a fragment thereof. In some embodiments, the Fas ligand-dependent inflammatory condition is glaucoma or cutaneous lupus.

Alternatively, the present disclosure provides compositions for use in methods for treating a Fas ligand-dependent inflammatory condition. In some embodiments, the methods involve detecting presence or absence of membrane-bound Fas ligand (mFasL) and/or soluble Fas ligand (sFasL) in a tissue of a subject, and treating the subject based on presence or absence of mFasL and/or sFasL. In some embodiments, the methods further involve treating the subject by administering to the subject an effective amount of recombinant adeno-associated virus (rAAV), wherein the rAAV comprises (i) capsid protein and (ii) a nucleic acid engineered to express sFasL or a fragment thereof.

In yet another aspect, the present disclosure provides methods for administering soluble FasL (sFasL) or a fragment thereof to a subject. In some embodiments, the subject has age-related elevated intraocular pressure. In some embodiments, the methods further nvolve intraocularly administering a recombinant adeno-associated virus (rAAV) that comprises a nucleic acid engineered to express sFasL or a fragment thereof.

In some embodiments, the subject to be treated in a method described herein is a patient (e.g., a human patient) who has or is suspected of having a Fas ligand-dependent inflammatory condition. In some examples, the subject is a human patient who has or is suspected of having glaucoma. Such a human patient may exhibit an elevated intraocular pressure (IOP), loss of retinal ganglion cells (RGCs) and/or axons, increased expression of pro-apoptotic genes (*e.g.*, Bax, FADD, Fas, and FasL), decreased expression of anti-apoptotic genes (*e.g.*, c-Flip, Bcl-2, and CIAP-2), activated retinal glial cells, increased TNFα activity, increased expression of mFasL, and decreased expression of sFasL.

In some embodiments, any of the subjects to be treated by methods described herein may have been treated with another glaucoma therapy (*e.g.*, eyedrops, oral medications, or surgery). In some embodiments, methods described herein may further comprise the use of another anti-glaucoma treatment.

Also provided in the disclosure are (a) pharmaceutical compositions for use treating glaucoma in a subject. In some embodiments, such pharmaceutical compositions comprise one or more rAAVs that comprise a nucleic acid engineered to express sFasL or a fragment thereof described herein and a pharmaceutically acceptable carrier. In some embodiments, uses of rAAVs that comprise a nucleic acid engineered to express sFasL or a fragment thereof in manufacturing a medicament for glaucoma treatment are also provided.

The details of one or more embodiments of the disclosure are set forth in the description below. Other features or advantages of the present disclosure will be apparent from the following drawings and detailed description of several aspects, and also from the appended claims.

### BRIEF DESCRIPTION OF THE DRAWINGS

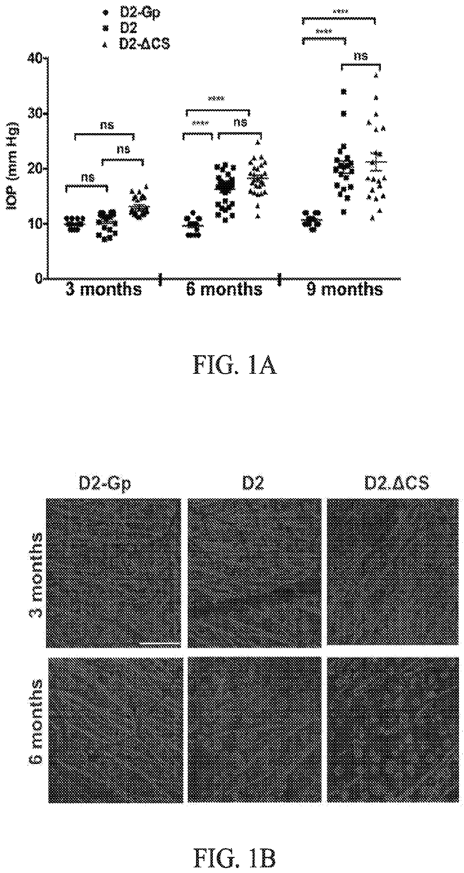

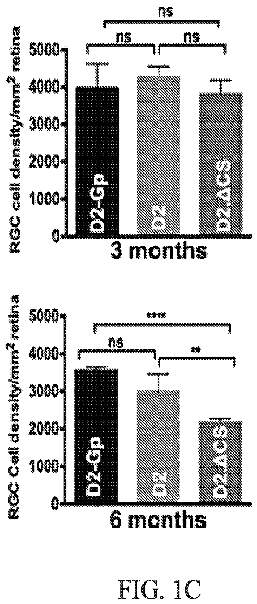

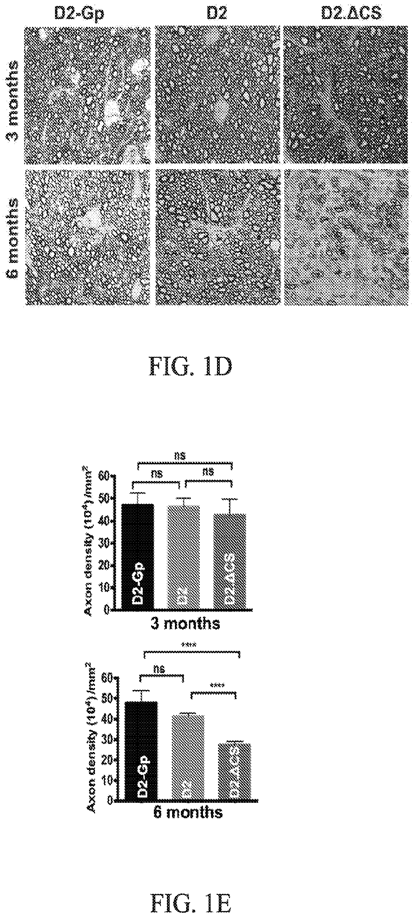

FIGs. 1A-1E show accelerated loss of RGCs and axons in DBA/2J mice that only express the membrane form of FasL. FIG. 1A shows IOP measurements were taken by rebound tonometry in D2-Gp, D2, and D2-ΔCS mice at 3, 6, and 9 months of age (N=16 D2-GP; N=22 D2, N=22 D2-ΔCS). Data is presented as mean IOP ± SEM. FIG. 1B shows representative confocal images of retinal flat-mounts isolated from D2-Gp, D2, and D2-ΔCS mice at 3 and 6 months of age, stained with β-III tubulin and RGC-specific marker and DAPI a nuclear stain, scale 75µm. FIG. 1C shows the quantification of β-III tubulin positive RGCs, represented as RGC density/mm² retina. N=10 eyes per group. FIG. 1D shows representative photomicrographs of PPD optic nerve cross sections taken from D2-Gp, D2 and D2-ΔCS at 3 and 6 months of age, Scale 100x. FIG. 1E shows the quantification of healthy axon, represented as axon density (10⁴)/mm². N=10 optic nerves per group. ns - non significant, *P<0.05, **P<0.01, ****P<0.0001.

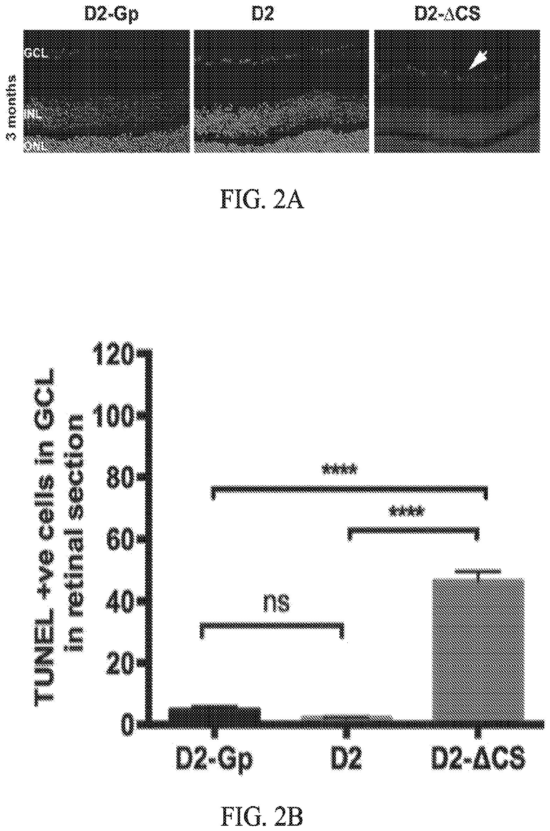

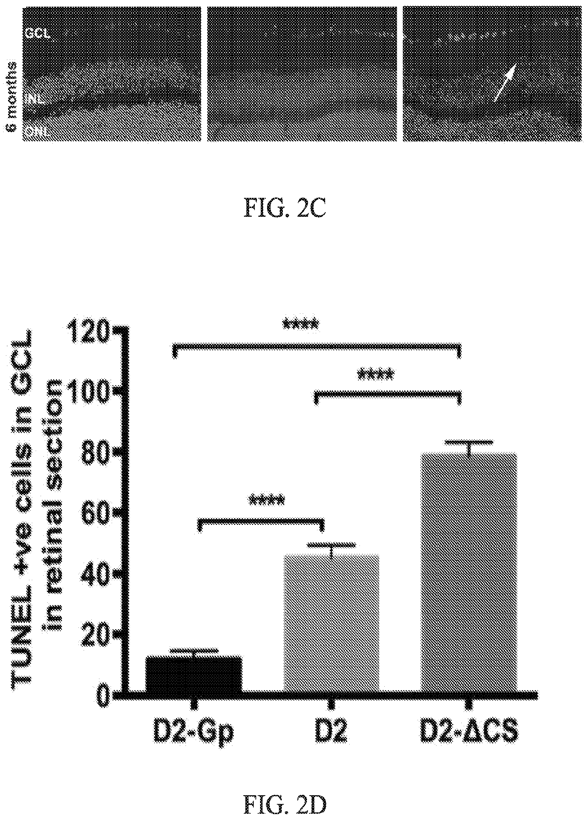

FIGs. 2A-2D show accelerated apoptosis coincides with RGC loss in the absence of sFasL. Representative TUNEL staining in paraffin embedded retinal sections taken from D2-Gp, D2 and D2-ΔCS mice at 3 months of age (FIG. 2A) and 6 months of age (FIG. 2C), scale 100µm. GCL, ganglion cells layer; INL, inner nuclear layer; ONL, outer nuclear layer; white arrowhead = TUNEL positive cells in GCL; white arrow = TUNEL positive cells in INL. TUNEL-positive cells in the GCL were quantitated at 3 months of age (FIG. 2B) and 6 months of age (FIG. 2D), represented as TUNEL positive cells/retinal sections (9 sections/retina), N=8 per group. ns-non significant, ***P<0.001, ****P<0.0001.

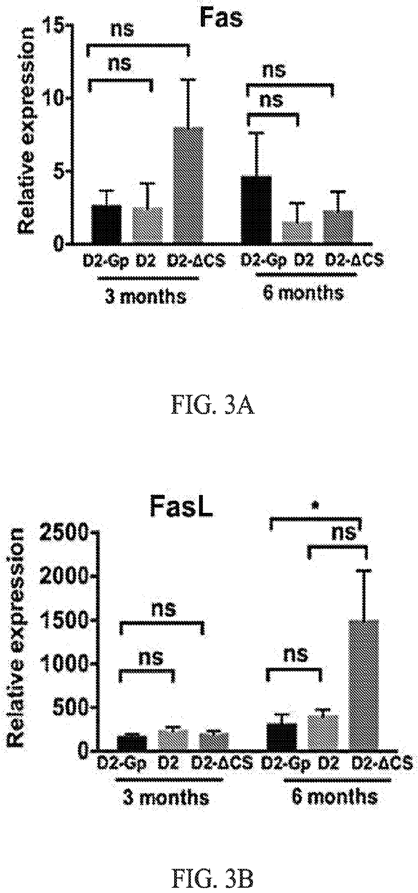

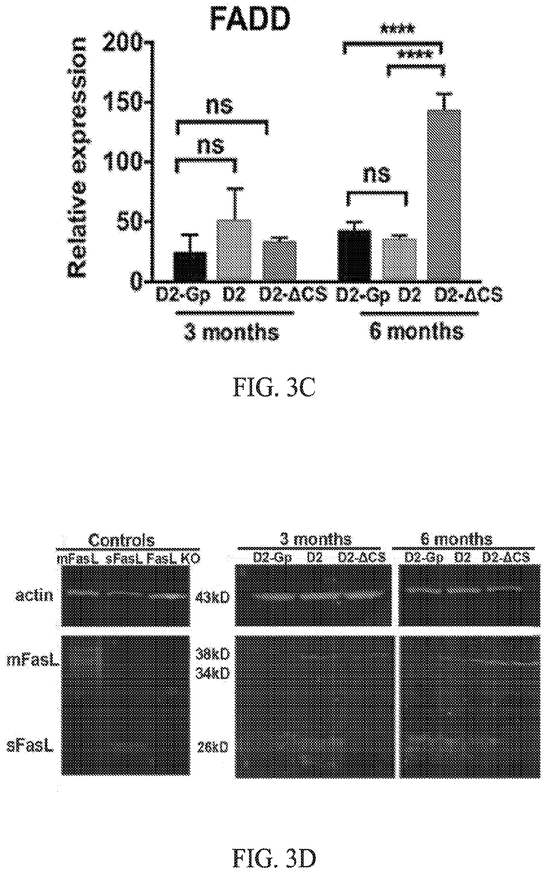

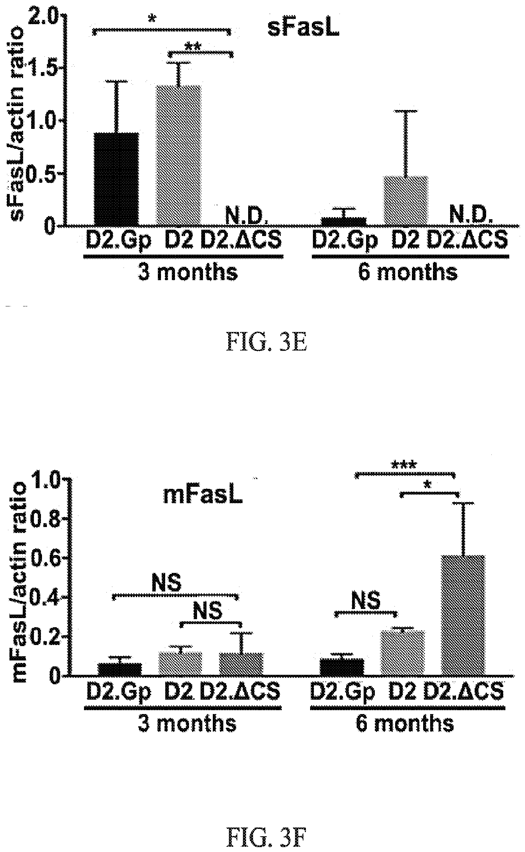

FIGs. 3A-3F show the expression of Fas, FasL, and FADD in D2-Gp, D2 and D2-ΔCS animals. Quantitative RT-PCR was performed on the neural retina isolated from D2-GP, D2, and D2-ΔCS mice at 3 and 6 months of age to quantitate mRNA levels of Fas (FIG. 3A), FasL (FIG. 3B), and FADD (FIG. 3C). N=6 per group. Representative Western blot from protein lysates (20µg/sample) prepared from posterior eye cups isolated from D2-GP, D2, and D2-ΔCS mice at 3 and 6 months (FIG. 3D). Protein lysates prepared from L5178Y-R tumor transfectants over expressing sFasL or mFasL were used as positive controls and a protein lysate prepared from a posterior eyecup isolated from FasL-KO mice was used as a negative control. Densitometry analysis of mFasL 34 and 38 kD bands (FIG. 3E) and sFasL 26 kD band (FIG. 3F) is the average of 3 independent experiments (3 independent blots consisting of 1 posterior segment per group, per experiment) Error bar indicate SEM+ ns-non significant. N.D.- not detected, *P<0.05,**P<0.01, ****P<0.0001.

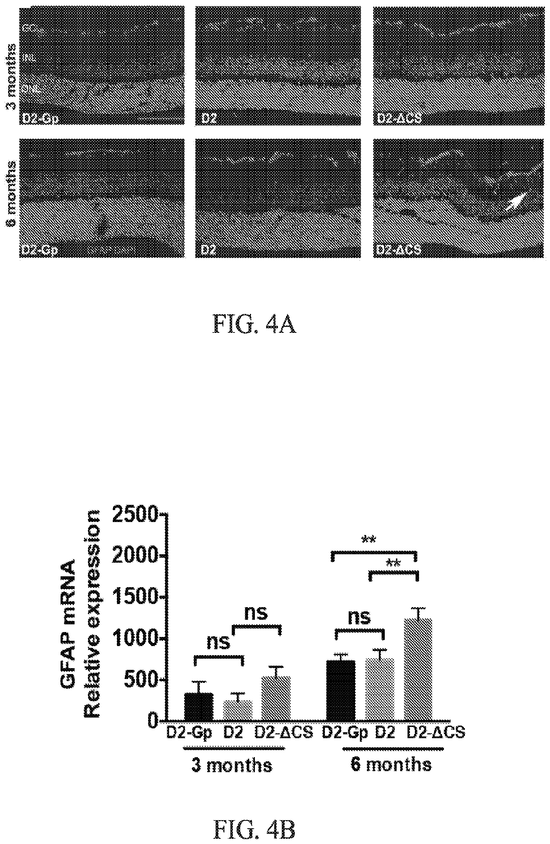

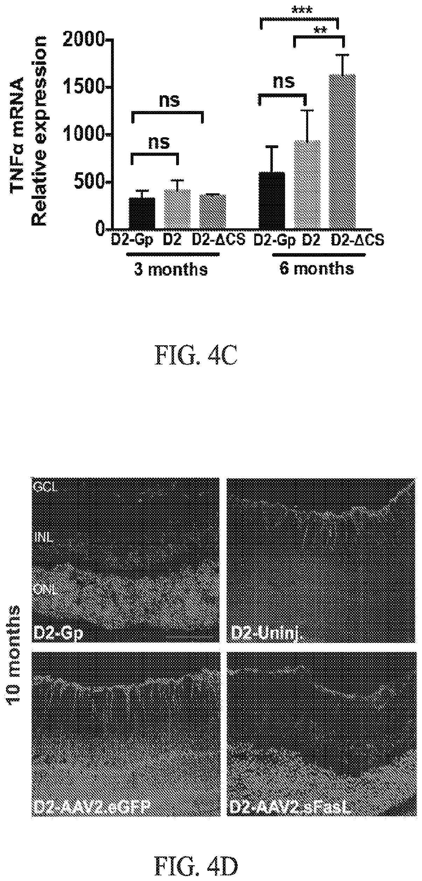

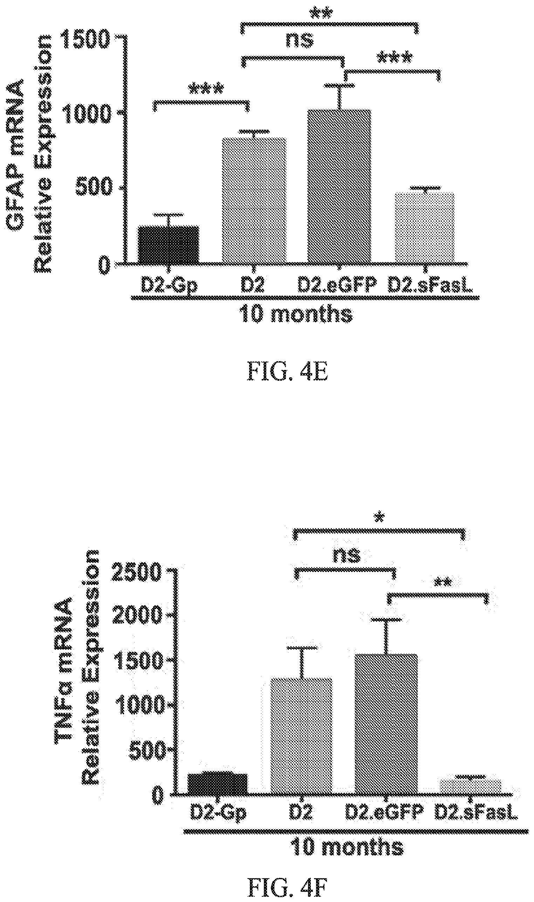

FIGs. 4A-4F show the expression of GFAP and TNFα in the retina of D2-ΔCS mice and D2 mice treated with AAV2.sFasL. Representative confocal microscopy images of paraffin embedded retinal sections taken from D2-Gp, D2, and D2-ΔCS mice at 3 months of age and 6 months of age and stained for GFAP and DAPI, white arrow=GFAP in muller cells, scale 100µm (FIG. 4A). Quantitative RT- PCR was performed on the neural retina isolated from D2-GP, D2, and D2-ΔCS mice at 3 and 6 months of age to quantitate mRNA levels of GFAP (FIG. 4B) and TNFα (FIG. 4C). Representative confocal microscopy images of paraffin embedded retinal sections taken from D2-Gp, D2-uninj. D2-AAV2-eGFP, and D2-AAV2-sFasL mice at 10 months of age and stained for GFAP and DAPI, scale 100µm (FIG. 4D). Quantitative RT- PCR was performed on the neural retina isolated from D2-Gp, D2-uninj., D2-AAV2-eGFP, and D2-AAV2-sFasL mice at 10 months of age to quantitate mRNA levels of GFAP (FIG. 4E) and TNFα (FIG. 4F). N=6 per group. Error bar indicates SEM. ns-non significant, *P<0.05, **<0.01, ****<0.0001.

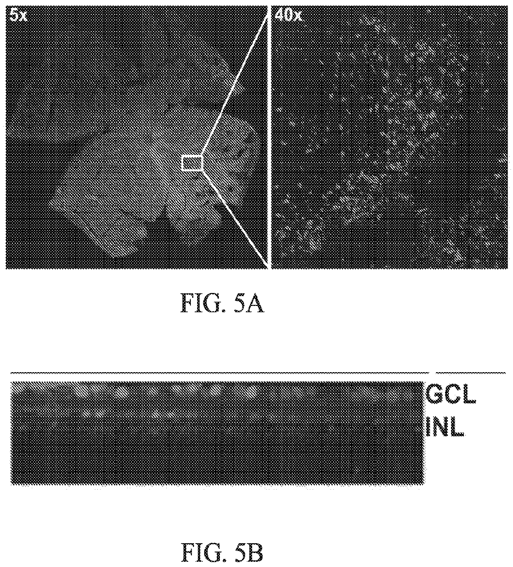

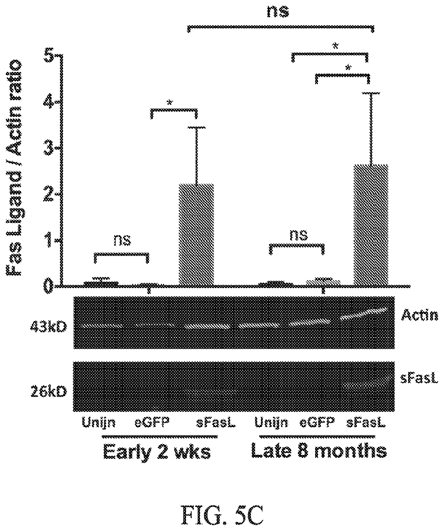

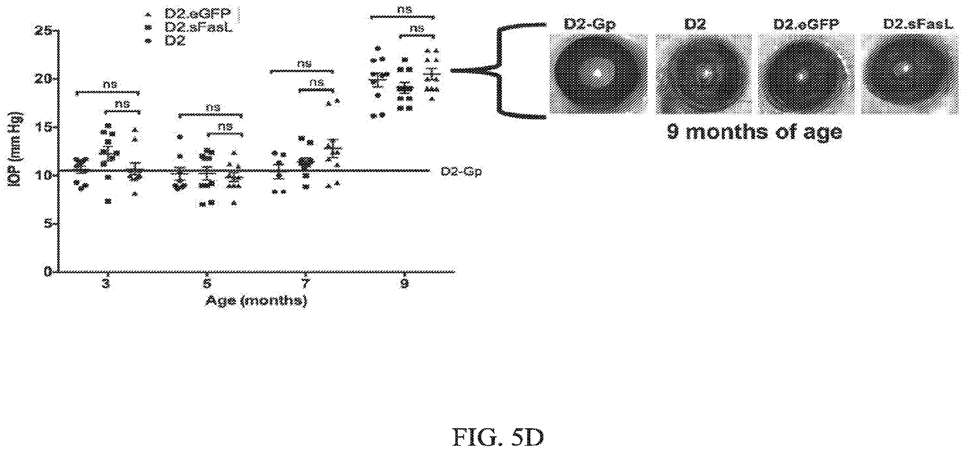

FIGs. 5A-5D shows the overexpression of sFasL in DBA2/J mice. DBA/2J mice received one intravitreal injection of AAV2.sFasL or AAV2.eGFP as a control at 2 months of age. Retinal flat-mount showing expression of AAV2.eGFP throughout the retina, scale 5x and 100µm (FIG. 5A). Cross sectional 3-D reconstruction of the retina (FIG. 5B). GCL-ganglion cell layer, INL-inner nuclear layer. Western blot form neural retina lysates (5µg/sample) showing overexpression of sFasL at 26 kD at both 2wk and at 8 months post AAV2 injection, N=3 per group (FIG. 5C). IOP measurements were taken at 3, 5, 7, and 9 months of age by rebound tonometry in D2 (uninjected), D2-AAV2.eGFP, and D2-AAV2.sFasL (FIG. 5D). Data is presented as mean IOP ± SEM IOP (N=10 per group). Pigment dispersion and iris stromal atrophy in D2-Gp, D2 (uninjected), D2-AAV.eGFP, and D2-AAV.sFasL at 9 months of age. *P<0.05.

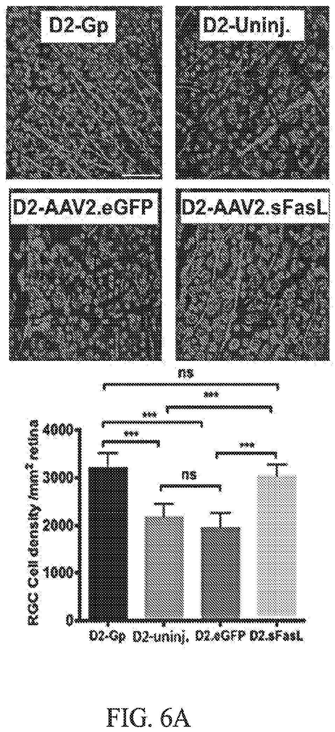

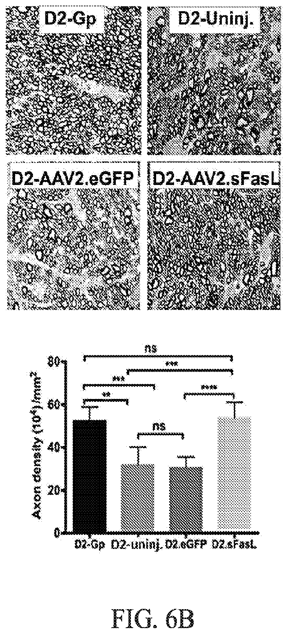

FIGs. 6A-6B show intravitreal AAV2.sFasL protects RGCs and axons in DBA/2J mice. DBA/2J mice received one intravitreal injection of AAV2.sFasL or AAV2.eGFP as a control at 2 months of age. At 10 months of age, AAV2.sFasL and AAV2.eGFP treated mice, in addition to age-matched D2-uninj (uninjected) and D2.Gp mice were euthanized and retinas and optic nerves processed for analysis of RGCs and axons. Representative confocal images of retinal flat-mounts isolated from D2-Gp, D2 uninjected, D2-AAV2.eGFP and D2-AAV2.sFasL mice, stained with β-III tubulin, a RGC-specific marker, and DAPI, scale 50µm. Quantification of β-III tubulin positive RGCs, represented as RGC density/mm² retina. N=10 eyes per group (FIG. 6A). Representative photomicrographs of PPD optic nerve cross sections taken from D2-Gp, D2 uninjected, D2-AAV2.eGFP and D2-AAV2.sFasL mice. Quantification of healthy axon, represented as axon density (10⁴)/mm². N=10 eyes per group (FIG. 6B). Scale 100x, ns- non significant, **P<0.01, ***P<0.001 ****P<0.0001

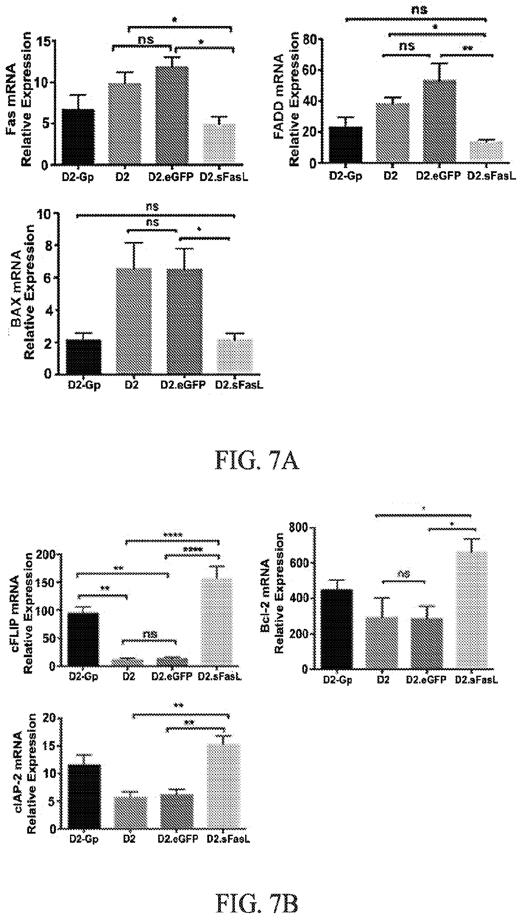

FIGs. 7A-7B show the expression of pro- and anti-apoptotic mediators in D2 mice treated with AAV2.sFasL. Quantitative RT- PCR was performed on the neural retina to quantitate mRNA levels of pro-apoptotic mediators (FIG. 7A) Fas, FADD, and BAX, and anti-apoptotic mediators cFLIP, Bcl2, and cIAP2 (FIG. 7B). N=5 per group. Error bar indicates SEM. ns-non significant, *P<0.05, **P<0.01, ****P<0.0001.

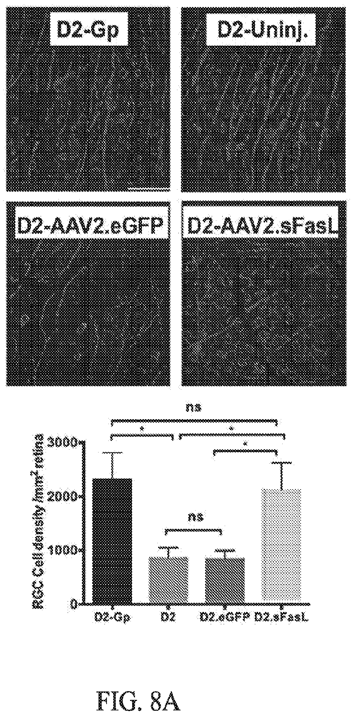

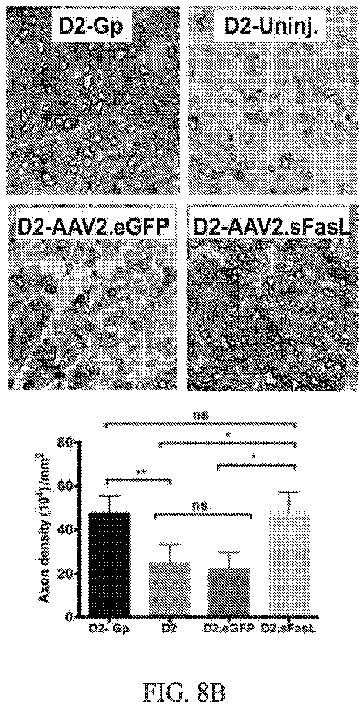

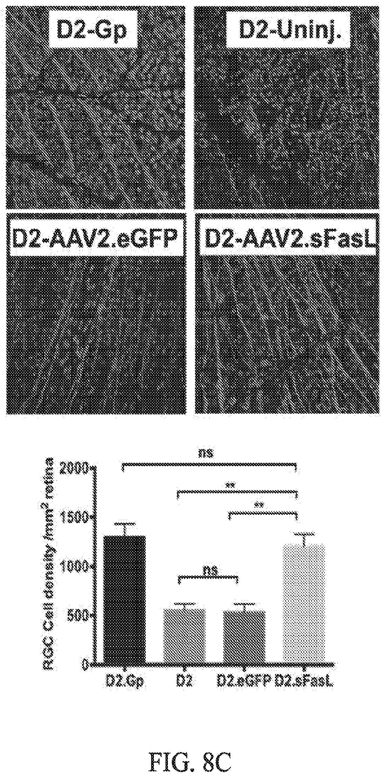

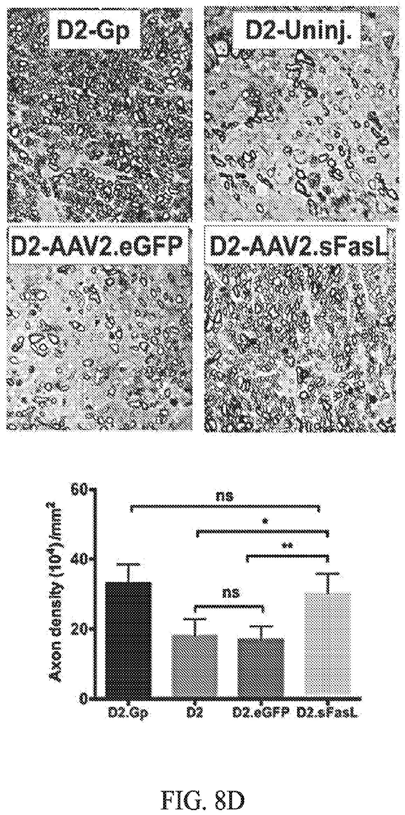

FIGs. 8A-8D show that treatment with AAV2.sFasL provides long-term protection of RGCs and axons in D2 mice when given before or after disease begins. Representative confocal microscopy images (scale 100µm) from retinal whole mounts stained with βIII tubulin and DAPI at 15 months of age from D2 mice treated with AAV2-eGFP or AAV2-sFasL at 2 months of age (Pre-treatment) (FIG. 8A) and D2 mice treated with AAV2-eGFP or AAV2-sFasL at 7 months of age (after disease has started) (FIG. 8C). Age matched D2-Gp and D2-uninjected mice served as controls, (scale 100µm). Quantification of β-III tubulin positive RGCs, represented as RGC density/mm² retina. Representative photomicrographs of optic nerve cross sections stained with PPD at 15 months of age from D2 mice treated with AAV2-eGFP or AAV2-sFasL at 2 months of age (FIG. 8B) and D2 mice treated with AAV2-eGFP or AAV2-sFAsL at 7 months of age (FIG. 8D). Scale 100x. Age matched D2-Gp and D2-uninjected mice served as controls. Quantification of healthy axon, represented as axon density (10⁴)/mm². N=10 eyes per group.ns- non significant, *P<0.05, **P<0.01, ****P<0.0001.

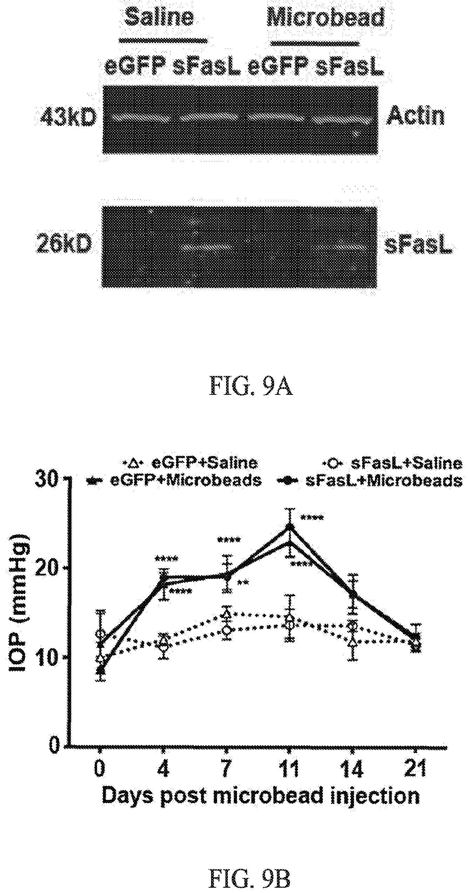

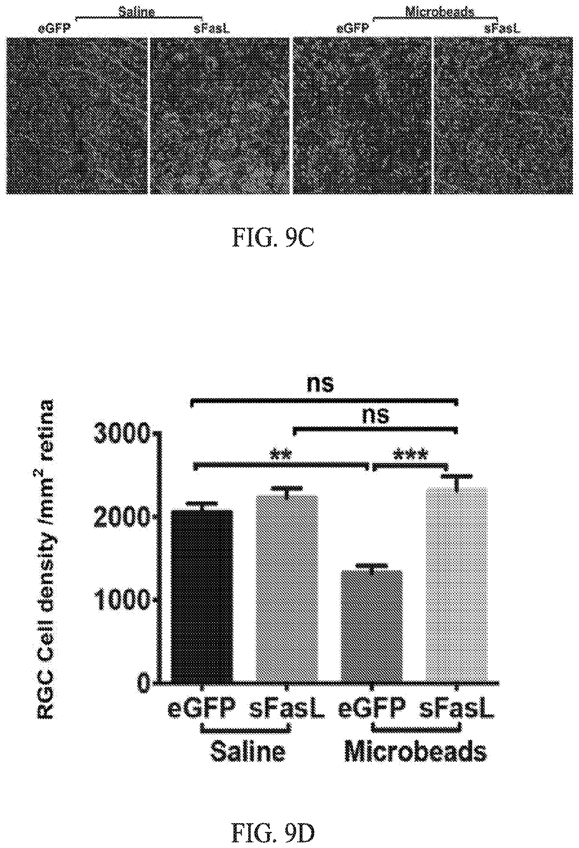

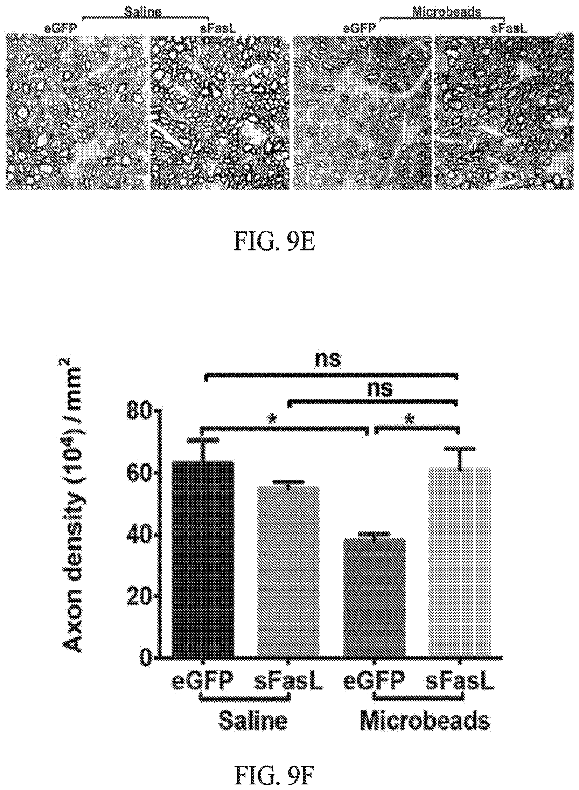

FIGs. 9A-9F show the pre-treatment with AAV2.sFasL protects RGC cell death in microbead-induced mouse model of elevated IOP in C57/BL6 mice. Western blot form neural retina lysates (5ug/sample) showing expression of sFasL at 26 kD and actin in Saline and Microbead injected B6.AAV2.eGFP and B6.AAV2.sFasL mice at 4 weeks post microbead or saline injections (FIG. 9A). IOP measurements were taken by rebound tonometry from B6.AAV2.eGFP and B6.AAV2.sFasL mice treated with saline or microbeads (FIG. 9B). Data is presented as mean IOP ± SEM, (N=10 per group). At 28 days post microbead injection the neural retina and optic nerve were processed for quantification of RGCs and axons. Representative confocal microscopy images from retinal flat-mounts stained with βIII tubulin, a RGC-specific marker, and DAPI at 4 weeks post microbead or saline injections (FIG. 9C). Quantification of β-III tubulin positive RGCs, represented as RGC density/mm² retina, scale 75µm (FIG. 9D). Representative photomicrographs of optic nerve cross sections stained with PPD at 4 weeks post microbead or saline injections, scale 100x (FIG. 9E). Quantification of healthy axon, represented as axon density (10⁴)/mm²(FIG. 9F). N=5 eyes per group. ns- non significant, *P<0.05, **P<0.01, ***P<0.001, ****P<0.0001.

### DETAILED DESCRIPTION

Aspects of the disclosure relate to certain protein-encoding transgenes (*e*.*g*., soluble Fas ligand (sFasL) or a fragment thereof) that when delivered to a subject are effective for treating or preventing retinal ganglion cell (RGC) death in the subject. Accordingly, methods and compositions described by the disclosure are useful, in some aspects, for the treatment of glaucoma.

### Methods for treating glaucoma

Methods for delivering a transgene (*e*.*g*., a gene encoding a sFasL protein or a fragment thereof) to a subject are provided by the disclosure. The methods typically involve administering to a subject an effective amount of an isolated nucleic acid encoding a sFasL protein fragment, or a rAAV comprising a nucleic acid for expressing a sFasL protein fragment.

Fas Ligand (FasL) is a 40 kDa type II transmembrane protein of the TNF family, originally identified by its capacity to induce apoptosis in Fas receptor positive cells. FasL can be expressed as a membrane-bound protein (mFasL), or cleaved and released as a soluble protein (sFasL).

The human *FasL* gene encodes for a 281 amino acid protein. In some aspects, the human *FasL* gene encodes a protein comprising the amino acid sequence set forth in SEQ ID NO: 6, and as described as GenBank Accession Number NM_000639.2. In some aspects, the human sFasL protein corresponding to amino acid residues 127-281 of human FasL is set forth as SEQ ID NO: 3. In some aspects, the human sFasL protein is encoded by nucleic acids set forth in SEQ ID NO: 2.

Aspects of the instant disclosure are based, in part, on the surprising discovery that sFasL prevents loss of RGCs and axons when expressed in a subject in need thereof, for example via administration of a viral vector (*e*.*g*., rAAV).

Accordingly in some aspects, the disclosure provides a transgene encoding a sFasL protein or a fragment thereof. A "sFasL protein fragment" refers to a functional 2 to 154 (*e.g.*, any integer between 2 and 154) amino acid portion of a sFasL protein. In some aspects, the sFasL protein fragment comprises a contiguous amino acid portion (*e*.*g*., amino acids 1 to 154) of sFasL (e*.g*., SEQ ID NO: 3). In some aspects, the sFasL protein fragment comprises one or more (*e.g.*, 2, 3, 4, 5, 6, 7, 8, 9, 10, or more) interrupted amino acid portions (*e.g.*, amino acids 1 to 10, 32 to 120 and 64 to 150) of sFasL (*e*.*g*., SEQ ID NO: 3)

In some aspects, the transgenes encoding a sFasL or fragment thereof described by the disclosure prevents loss of RGCs and axons, and are therefore useful for treating glaucoma. Generally, "glaucoma" refers to a group of eye conditions that damage the optic nerve. In some aspects, damage to the optic nerve in glaucoma is caused by elevated pressure in the eye. Examples of glaucoma include, but are not limited to, open-angle glaucoma, angle-closure glaucoma, normal-tension glaucoma (NTG), congenital glaucoma, secondary glaucoma, pigmentary glaucoma, pseudoexfoliative glaucoma, traumatic glaucoma, neovascular glaucoma, irido corneal endothelial syndrome (ICE), and uveitic glaucoma.

In some aspects, the disclosure provides a composition for use in method for treating glaucoma in a subject in need thereof, the method comprising administering to a subject having glaucoma a therapeutically effective amount of an isolated nucleic acid, or a rAAV, as described by the disclosure.

An "effective amount" of a substance is an amount sufficient to produce a desired effect. In some aspects, an effective amount of an isolated nucleic acid (*e.g.*, an isolated nucleic acid comprising a transgene encoding a sFasL protein or fragment thereof as described herein) is an amount sufficient to transfect (or infect in the context of rAAV mediated delivery) a sufficient number of target cells of a target tissue of a subject. In some aspects, a target tissue is ocular tissue (*e*.*g*., photoreceptor cells, rod cells, cone cells, retinal ganglion cells, retinal cells, *etc.*)*.* In some aspects, an effective amount of an isolated nucleic acid (*e.g.*, which may be delivered via an rAAV) may be an amount sufficient to have a therapeutic benefit in a subject, *e.g.*, to increase or supplement the expression of a gene or protein of interest (*e*.*g*., sFasL), or to improve in the subject one or more symptoms of disease (*e.g.,* a symptom of glaucoma, such as RGC damage), *etc.* The effective amount will depend on a variety of factors such as, for example, the species, age, weight, health of the subject, and the tissue to be targeted, and may thus vary among subject and tissue as described elsewhere in the disclosure.

As used herein, the term "treating" refers to the application or administration of a composition comprising sFasL or a fragment thereof to a subject, who has glaucoma, a symptom of glaucoma, or a predisposition toward glaucoma, with the purpose to cure, heal, alleviate, relieve, alter, remedy, ameliorate, improve, or affect the disorder, the symptom of the disease, or the predisposition toward glaucoma.

Alleviating glaucoma includes delaying the development or progression of the disease, or reducing disease severity. Alleviating the disease does not necessarily require curative results. As used therein, "delaying" the development of a disease (such as glaucoma) means to defer, hinder, slow, retard, stabilize, and/or postpone progression of the disease. This delay can be of varying lengths of time, depending on the history of the disease and/or individuals being treated. A method that "delays" or alleviates the development of a disease, or delays the onset of the disease, is a method that reduces probability of developing one or more symptoms of the disease in a given time frame and/or reduces extent of the symptoms in a given time frame, when compared to not using the method. Such comparisons are typically based on clinical studies, using a number of subjects sufficient to give a statistically significant result.

"Development" or "progression" of a disease means initial manifestations and/or ensuing progression of the disease. Development of the disease can be detectable and assessed using standard clinical techniques as well known in the art. However, development also refers to progression that may be undetectable. For purpose of this disclosure, development or progression refers to the biological course of the symptoms. "Development" includes occurrence, recurrence, and onset. As used herein "onset" or "occurrence" of a glaucoma includes initial onset and/or recurrence.

In some embodiments, sFasL or a fragment thereof is to be administered to a subject in need of the treatment at an amount sufficient for lowering intraocular pressure in the subject by at least 5% (*e.g.*, 5%, 10%, 20%, 30%, 40%, 50%, 60%, 70%, 80%, 90%, or greater). In some embodiments, sFasL or a fragment thereof is to be administered to a subject in need of the treatment at an amount sufficient for inactivating retinal glial cells in the subject by at least 5% (*e.g.*, 5%, 10%, 20%, 30%, 40%, 50%, 60%, 70%, 80%, 90%, or greater). In some aspects, sFasL or a fragment thereof is to be administered to a subject in need of the treatment at an amount sufficient for inhibiting TNFα activity in the subject by at least 5% (*e.g.*, 5%, 10%, 20%, 30%, 40%, 50%, 60%, 70%, 80%, 90%, or greater). In some aspects, sFasL or a fragment thereof is to be administered to a subject in need of the treatment at an amount sufficient for reducing retinal ganglion cell (RGC) death and/or reducing axonal degeneration in the subject by at least 5% (*e.g.*, 5%, 10%, 20%, 30%, 40%, 50%, 60%, 70%, 80%, 90%, or greater).

Methods and compositions for treating Fas ligand-dependent inflammatory conditions are also provided herein. As used herein, a "Fas ligand-dependent inflammatory condition" is a condition or a disease mediated by Fas ligand-dependent inflammation. Fas ligand-dependent inflammation may may be involved in ocular inflammatory diseases. Non-limiting examples of ocular inflammatory diseases include, but are not limited to, allergic conjunctivitis, uveitis, scleritis, episcleritis, optic neuritis, keratitis, orbital pseudotumor, retinal vasculitis, chronic conjunctivitis, and autoimmunity induced chronic inflammation (e.g., rheumatoid arthritis, systemic lupus erythematosus). Often, Fas ligand-dependent inflammatory conditions are linked to inflammation of the eye caused by mFasL. Without wishing to be bound by any particular theory, rAAV-based delivery of sFasL reduces inflammation of the eye in subjects having a Fas ligand-dependent inflammatory condition.

In some aspects, the method for treating a Fas ligand-dependent inflammatory condition comprises administering to a subject in need thereof an effective amount of recombinant adeno-associated virus (rAAV), wherein the rAAV comprises (i) capsid protein and (ii) a nucleic acid engineered to express sFasL or a fragment thereof.

In some aspects, the method for treating a Fas ligand-dependent inflammatory condition, comprises detecting presence or absence of membrane-bound Fas ligand (mFasL) and/or soluble Fas ligand (sFasL) in a tissue of a subject, and treating the subject based on presence or absence of mFasL and/or sFasL, wherein treating the subject comprises administering to a subject in need thereof an effective amount of recombinant adeno-associated virus (rAAV), wherein the rAAV comprises (i) capsid protein and (ii) a nucleic acid engineered to express sFasL or a fragment thereof.

The skilled artisan recognizes that FasL proteins (*e.g.*, FasL, mFasL, or sFasL) may be detected by any method known in the art. In some aspects FasL proteins are detecting using electrophoresis, Western blot, and mass spectrometry. In some aspects, FasL proteins are detected in ocular fluids (*e.g.*, intraocular fluid, aqueous humor, or tears).

### Combination therapy

Also provided herein are combined therapies using sFasL or a fragment thereof described herein and another anti-glaucoma therapeutic agent, such as those described herein. The term combination therapy, as used herein, embraces administration of these agents (*e.g.*, sFasL or a fragment thereof and an anti-glaucoma therapeutic agent) in a sequential manner, that is, wherein each therapeutic agent is administered at a different time, as well as administration of these therapeutic agents, or at least two of the agents, in a substantially simultaneous manner.

Sequential or substantially simultaneous administration of each agent can be affected by any appropriate route including, but not limited to, rAAV-mediated delivery, oral routes, intravenous routes, intramuscular, subcutaneous routes, and direct absorption through mucous membrane tissues. The agents can be administered by the same route or by different routes. For example, a first agent (*e.g.*, sFasL or a fragment thereof) can be administered via rAAV-mediated delivery, and a second agent (*e.g.*, an anti-glaucoma agent) can be administered orally.

As used herein, the term "sequential" means, unless otherwise specified, characterized by a regular sequence or order, *e.g.*, if a dosage regimen includes the administration of sFasL or a fragment thereof and an anti-glaucoma agent, a sequential dosage regimen could include administration of sFasL or a fragment thereof before, simultaneously, substantially simultaneously, or after administration of the anti-glaucoma agent, but both agents will be administered in a regular sequence or order. The term "separate" means, unless otherwise specified, to keep apart one from the other. The term "simultaneously" means, unless otherwise specified, happening or done at the same time, *i.e.*, the agents of the disclosure are administered at the same time. The term "substantially simultaneously" means that the agents are administered within minutes of each other (*e.g.*, within 10 minutes of each other) and intends to embrace joint administration as well as consecutive administration, but if the administration is consecutive it is separated in time for only a short period (*e.g.*, the time it would take a medical practitioner to administer two agents separately). As used herein, concurrent administration and substantially simultaneous administration are used interchangeably. Sequential administration refers to temporally separated administration of the agents described herein.

Combination therapy can also embrace the administration of the agents described herein (*e.g.*, sFasL or a fragment thereof and an anti-glaucoma agent) in further combination with other biologically active ingredients (*e.g.*, a different anti-glaucoma agent) and non-drug therapies (*e.g.*, surgery).

It should be appreciated that any combination of sFasL or a fragment thereof and another anti-glaucoma agent (*e.g.*, delivered via eyedrops) may be used in any sequence for treating a glaucoma. The combinations described herein may be selected on the basis of a number of factors, which include but are not limited to the effectiveness of reducing glaucoma disease progression, lowering intraocular pressure (IOP), inactivating retinal glial cells, inhibiting TNFα activity, reducing retinal ganglion cell (RGC) death, reducing axonal degeneration, and/or alleviating at least one symptom associated with the glaucoma, or the effectiveness for mitigating the side effects of another agent of the combination. For example, a combined therapy described herein may reduce any of the side effects associated with each individual members of the combination, for example, a side effect associated with the anti-glaucoma agent.

In some aspects, another anti-glaucoma therapeutic agent is eyedrops, an oral medication, and/or a surgical therapy. Eyedrops may comprise one or more therapeutic agents, for example, prostaglandins, beta blockers, alpha-adrenergic agonists, carbonic anhydrase inhibitors, miotic agents, and cholinergic agents. In some aspects, the oral medication comprises a carbonic anhydrase inhibitor. Examples of a surgical therapy include, but are not limited to, laser therapy, filtering surgery, drainage tubes, and electrocautery.

### Isolated nucleic acids

In some aspects, the disclosure provides isolated nucleic acids that are useful for expressing human sFasL, or a fragment thereof. A "nucleic acid" sequence refers to a DNA or RNA sequence. In some aspects, proteins and nucleic acids of the disclosure are isolated. As used herein, the term "isolated" means artificially produced. As used herein with respect to nucleic acids, the term "isolated" means: (i) amplified *in vitro* by, for example, polymerase chain reaction (PCR); (ii) recombinantly produced by cloning; (iii) purified, as by cleavage and gel separation; or (iv) synthesized by, for example, chemical synthesis. An isolated nucleic acid is one which is readily manipulable by recombinant DNA techniques well known in the art. Thus, a nucleotide sequence contained in a vector in which 5' and 3' restriction sites are known or for which polymerase chain reaction (PCR) primer sequences have been disclosed is considered isolated but a nucleic acid sequence existing in its native state in its natural host is not. An isolated nucleic acid may be substantially purified, but need not be. For example, a nucleic acid that is isolated within a cloning or expression vector is not pure in that it may comprise only a tiny percentage of the material in the cell in which it resides. Such a nucleic acid is isolated, however, as the term is used herein because it is readily manipulable by standard techniques known to those of ordinary skill in the art. As used herein with respect to proteins or peptides, the term "isolated" refers to a protein or peptide that has been isolated from its natural environment or artificially produced (*e.g.*, by chemical synthesis, by recombinant DNA technology, *etc.*)*.*

The skilled artisan will also realize that conservative amino acid substitutions may be made to provide functionally equivalent variants, or homologs of the capsid proteins. In some aspects the disclosure embraces sequence alterations that result in conservative amino acid substitutions. As used herein, a conservative amino acid substitution refers to an amino acid substitution that does not alter the relative charge or size characteristics of the protein in which the amino acid substitution is made. Variants can be prepared according to methods for altering polypeptide sequence known to one of ordinary skill in the art such as are found in references that compile such methods, *e.g.*, Molecular Cloning: A Laboratory Manual, J. Sambrook, et al., eds., Second Edition, Cold Spring Harbor Laboratory Press, Cold Spring Harbor, New York, 1989, or Current Protocols in Molecular Biology, F.M. Ausubel, et al., eds., John Wiley & Sons, Inc., New York. Conservative substitutions of amino acids include substitutions made among amino acids within the following groups: (a) M, I, L, V; (b) F, Y, W; (c) K, R, H; (d) A, G; (e) S, T; (f) Q, N; and (g) E, D. Therefore, one can make conservative amino acid substitutions to the amino acid sequence of the proteins and polypeptides disclosed herein.

The isolated nucleic acids of the disclosure may be recombinant adeno-associated virus (AAV) vectors (rAAV vectors). In some aspects, an isolated nucleic acid as described by the disclosure comprises a region (e.g., a first region) comprising a first adeno-associated virus (AAV) inverted terminal repeat (ITR), or a variant thereof. The isolated nucleic acid (*e.g.*, the recombinant AAV vector) may be packaged into a capsid protein and administered to a subject and/or delivered to a selected target cell. "Recombinant AAV (rAAV) vectors" are typically composed of, at a minimum, a transgene and its regulatory sequences, and 5' and 3' AAV inverted terminal repeats (ITRs). The transgene may comprise, as disclosed elsewhere herein, one or more regions that encode one or more proteins (*e*.*g*., human sFasL, or a fragment thereof). The transgene may also comprise a region encoding, for example, a miRNA binding site, and/or an expression control sequence (*e.g.*, a poly-A tail), as described elsewhere in the disclosure.

Generally, ITR sequences are about 145 bp in length. Preferably, substantially the entire sequences encoding the ITRs are used in the molecule, although some degree of minor modification of these sequences is permissible. The ability to modify these ITR sequences is within the skill of the art. (See, *e.g.*, texts such as Sambrook et al., "Molecular Cloning. A Laboratory Manual", 2d ed., Cold Spring Harbor Laboratory, New York (1989); and K. Fisher et al., J Virol., 70:520 532 (1996)). An example of such a molecule employed in the present disclosure is a "cis-acting" plasmid containing the transgene, in which the selected transgene sequence and associated regulatory elements are flanked by the 5' and 3' AAV ITR sequences. The AAV ITR sequences may be obtained from any known AAV, including presently identified mammalian AAV types. In some aspects of the disclosure, the isolated nucleic acid (*e.g.*, the rAAV vector) comprises at least one ITR having a serotype selected from AAV1, AAV2, AAV5, AAV6, AAV6.2, AAV7, AAV8, AAV9, AAV10, AAV11, and variants thereof. In some aspects of the disclosure, the isolated nucleic acid comprises a region (*e.g.*, a first region) encoding an AAV2 ITR.

In some aspects, the isolated nucleic acid further comprises a region (*e.g.*, a second region, a third region, a fourth region, *etc.*) comprising a second AAV ITR. In some aspects, the second AAV ITR has a serotype selected from AAV1, AAV2, AAV5, AAV6, AAV6.2, AAV7, AAV8, AAV9, AAV10, AAV11, and variants thereof. In some aspects, the second ITR is a mutant ITR that lacks a functional terminal resolution site (TRS). The term "lacking a terminal resolution site" can refer to an AAV ITR that comprises a mutation (*e*.*g*., a sense mutation such as a non-synonymous mutation, or missense mutation) that abrogates the function of the terminal resolution site (TRS) of the ITR, or to a truncated AAV ITR that lacks a nucleic acid sequence encoding a functional TRS (*e*.*g*., a ΔTRS ITR). Without wishing to be bound by any particular theory, a rAAV vector comprising an ITR lacking a functional TRS produces a self-complementary rAAV vector, for example as described by McCarthy (2008) Molecular Therapy 16(10):1648-1656.

In addition to the major elements identified above for the recombinant AAV vector, the vector also includes conventional control elements which are operably linked with elements of the transgene in a manner that permits its transcription, translation and/or expression in a cell transfected with the vector or infected with the virus produced by the disclosure. As used herein, "operably linked" sequences include both expression control sequences that are contiguous with the gene of interest and expression control sequences that act in trans or at a distance to control the gene of interest. Expression control sequences include appropriate transcription initiation, termination, promoter and enhancer sequences; efficient RNA processing signals such as splicing and polyadenylation (polyA) signals; sequences that stabilize cytoplasmic mRNA; sequences that enhance translation efficiency (*i.e.*, Kozak consensus sequence); sequences that enhance protein stability; and when desired, sequences that enhance secretion of the encoded product. A number of expression control sequences, including promoters which are native, constitutive, inducible and/or tissue-specific, are known in the art and may be utilized.

As used herein, a nucleic acid sequence (*e*.*g*., coding sequence) and regulatory sequences are said to be operably linked when they are covalently linked in such a way as to place the expression or transcription of the nucleic acid sequence under the influence or control of the regulatory sequences. If it is desired that the nucleic acid sequences be translated into a functional protein, two DNA sequences are said to be operably linked if induction of a promoter in the 5' regulatory sequences results in the transcription of the coding sequence and if the nature of the linkage between the two DNA sequences does not (1) result in the introduction of a frame-shift mutation, (2) interfere with the ability of the promoter region to direct the transcription of the coding sequences, or (3) interfere with the ability of the corresponding RNA transcript to be translated into a protein. Thus, a promoter region would be operably linked to a nucleic acid sequence if the promoter region were capable of effecting transcription of that DNA sequence such that the resulting transcript might be translated into the desired protein or polypeptide. Similarly two or more coding regions are operably linked when they are linked in such a way that their transcription from a common promoter results in the expression of two or more proteins having been translated in frame. In some aspects, operably linked coding sequences yield a fusion protein. In some aspects, operably linked coding sequences yield a functional RNA (*e*.*g*., miRNA).

A "promoter" refers to a DNA sequence recognized by the synthetic machinery of the cell, or introduced synthetic machinery, required to initiate the specific transcription of a gene. The phrases "operatively positioned," "under control" or "under transcriptional control" means that the promoter is in the correct location and orientation in relation to the nucleic acid to control RNA polymerase initiation and expression of the gene.

For nucleic acids encoding proteins, a polyadenylation sequence generally is inserted following the transgene sequences and before the 3' AAV ITR sequence. A rAAV construct useful in the present disclosure may also contain an intron, desirably located between the promoter/enhancer sequence and the transgene. One possible intron sequence is derived from SV-40, and is referred to as the SV-40 T intron sequence. Another vector element that may be used is an internal ribosome entry site (IRES). An IRES sequence is used to produce more than one polypeptide from a single gene transcript. An IRES sequence would be used to produce a protein that contain more than one polypeptide chains. Selection of these and other common vector elements are conventional and many such sequences are available [see, *e.g.*, Sambrook et al., and references cited therein at, for example, pages 3.18 3.26 and 16.17 16.27 and Ausubel et al., Current Protocols in Molecular Biology, John Wiley & Sons, New York, 1989]. In some aspects, a Foot and Mouth Disease Virus 2A sequence is included in polyprotein; this is a small peptide (approximately 18 amino acids in length) that has been shown to mediate the cleavage of polyproteins (Ryan, M D et al., EMBO, 1994; 4: 928-933; Mattion, N M et al., J Virology, November 1996; p. 8124-8127; Furler, S et al., Gene Therapy, 2001; 8: 864-873; and Halpin, C et al., The Plant Journal, 1999; 4: 453-459). The cleavage activity of the 2A sequence has previously been demonstrated in artificial systems including plasmids and gene therapy vectors (AAV and retroviruses) (Ryan, M D et al., EMBO, 1994; 4: 928-933; Mattion, N M et al., J Virology, November 1996; p. 8124-8127; Furler, S et al., Gene Therapy, 2001; 8: 864-873; and Halpin, C et al., The Plant Journal, 1999; 4: 453-459; de Felipe, P et al., Gene Therapy, 1999; 6: 198-208; de Felipe, P et al., Human Gene Therapy, 2000; 11: 1921-1931.; and Klump, H et al., Gene Therapy, 2001; 8: 811-817).

Examples of constitutive promoters include, without limitation, the retroviral Rous sarcoma virus (RSV) LTR promoter (optionally with the RSV enhancer), the cytomegalovirus (CMV) promoter (optionally with the CMV enhancer) [see, *e.g.*, Boshart et al., Cell, 41:521-530 (1985)], the SV40 promoter, the dihydrofolate reductase promoter, the β-actin promoter, the phosphoglycerol kinase (PGK) promoter, and the EF1α promoter [Invitrogen]. In some aspects, a promoter is an enhanced chicken β-actin promoter. In some aspects, a promoter is a U6 promoter. In some aspects, a promoter is a chicken beta-actin (CBA) promoter.

Inducible promoters allow regulation of gene expression and can be regulated by exogenously supplied compounds, environmental factors such as temperature, or the presence of a specific physiological state, *e.g.*, acute phase, a particular differentiation state of the cell, or in replicating cells only. Inducible promoters and inducible systems are available from a variety of commercial sources, including, without limitation, Invitrogen, Clontech and Ariad. Many other systems have been described and can be readily selected by one of skill in the art. Examples of inducible promoters regulated by exogenously supplied promoters include the zinc-inducible sheep metallothionine (MT) promoter, the dexamethasone (Dex)-inducible mouse mammary tumor virus (MMTV) promoter, the T7 polymerase promoter system (WO 98/10088); the ecdysone insect promoter (No et al., Proc. Natl. Acad. Sci. USA, 93:3346-3351 (1996)), the tetracycline-repressible system (Gossen et al., Proc. Natl. Acad. Sci. USA, 89:5547-5551 (1992)), the tetracycline-inducible system (Gossen et al., Science, 268:1766-1769 (1995), see also Harvey et al., Curr. Opin. Chem. Biol., 2:512-518 (1998)), the RU486-inducible system (Wang et al., Nat. Biotech., 15:239-243 (1997) and Wang et al., Gene Ther., 4:432-441 (1997)) and the rapamycin-inducible system (Magari et al., J. Clin. Invest., 100:2865-2872 (1997)). Still other types of inducible promoters which may be useful in this context are those which are regulated by a specific physiological state, *e*.*g*., temperature, acute phase, a particular differentiation state of the cell, or in replicating cells only.

In another aspect, the native promoter for the transgene will be used. The native promoter may be preferred when it is desired that expression of the transgene should mimic the native expression. The native promoter may be used when expression of the transgene must be regulated temporally or developmentally, or in a tissue-specific manner, or in response to specific transcriptional stimuli. In a further aspect, other native expression control elements, such as enhancer elements, polyadenylation sites or Kozak consensus sequences may also be used to mimic the native expression.

In some aspects, the regulatory sequences impart tissue-specific gene expression capabilities. In some cases, the tissue-specific regulatory sequences bind tissue-specific transcription factors that induce transcription in a tissue specific manner. Such tissue-specific regulatory sequences (*e.g.*, promoters, enhancers, *etc*..) are well known in the art. In some aspects, the tissue-specific promoter is an eye-specific promoter. Examples of eye-specific promoters include but are not limited to a retinoschisin promoter, K12 promoter, a rhodopsin promoter, a rod-specific promoter, a cone-specific promoter, a rhodopsin kinase promoter, a GRK1 promoter, an interphotoreceptor retinoid-binding protein proximal (IRBP) promoter, and an opsin promoter (*e.g.*, a red opsin promoter, a blue opsin promoter, *etc.).*

### Recombinant adeno-associated viruses (rAAVs)

In some aspects, the disclosure provides isolated AAVs. As used herein with respect to AAVs, the term "isolated" refers to an AAV that has been artificially produced or obtained. Isolated AAVs may be produced using recombinant methods. Such AAVs are referred to herein as "recombinant AAVs". Recombinant AAVs (rAAVs) preferably have tissue-specific targeting capabilities, such that a transgene of the rAAV will be delivered specifically to one or more predetermined tissue(s). The AAV capsid is an important element in determining these tissue-specific targeting capabilities. Thus, an rAAV having a capsid appropriate for the tissue being targeted can be selected.

Methods for obtaining recombinant AAVs having a desired capsid protein are well known in the art. (See, for example, US 2003/0138772). Typically the methods involve culturing a host cell which contains a nucleic acid sequence encoding an AAV capsid protein; a functional *rep* gene; a recombinant AAV vector composed of, AAV inverted terminal repeats (ITRs) and a transgene; and sufficient helper functions to permit packaging of the recombinant AAV vector into the AAV capsid proteins. In some aspects, capsid proteins are structural proteins encoded by the cap gene of an AAV. AAVs comprise three capsid proteins, virion proteins 1 to 3 (named VP1, VP2 and VP3), all of which are transcribed from a single cap gene via alternative splicing. In some aspects, the molecular weights of VP1, VP2 and VP3 are respectively about 87 kDa, about 72 kDa and about 62 kDa. In some aspects, upon translation, capsid proteins form a spherical 60-mer protein shell around the viral genome. In some aspects, the functions of the capsid proteins are to protect the viral genome, deliver the genome and interact with the host. In some aspects, capsid proteins deliver the viral genome to a host in a tissue specific manner.

In some aspects of the disclosure, an AAV capsid protein is of an AAV serotype selected from the group consisting of AAV2, AAV3, AAV4, AAV5, AAV6, AAV8, AAVrh8, AAV9, and AAV10. In some aspects, an AAV capsid protein is of a serotype derived from a non-human primate, for example AAVrh8 serotype. In some aspects, the AAV capsid protein is of a serotype that has tropism for the eye of a subject, for example an AAV (*e*.*g*., AAV5, AAV6, AAV6.2, AAV7, AAV8, AAV9, AAVrh.8, AAVrh.10, AAVrh.39 and AAVrh.43) that transduces ocular cells of a subject more efficiently than other vectors. In some aspects, an AAV capsid protein is of an AAV8 serotype or an AAV5 serotype. In some aspects, the AAV capsid protein comprises the sequence set forth in SEQ ID NO: 1.

The components to be cultured in the host cell to package a rAAV vector in an AAV capsid may be provided to the host cell in *trans.* Alternatively, any one or more of the required components (*e*.*g*., recombinant AAV vector, rep sequences, cap sequences, and/or helper functions) may be provided by a stable host cell which has been engineered to contain one or more of the required components using methods known to those of skill in the art. Most suitably, such a stable host cell will contain the required component(s) under the control of an inducible promoter. However, the required component(s) may be under the control of a constitutive promoter. Examples of suitable inducible and constitutive promoters are provided herein, in the discussion of regulatory elements suitable for use with the transgene. In still another alternative, a selected stable host cell may contain selected component(s) under the control of a constitutive promoter and other selected component(s) under the control of one or more inducible promoters. For example, a stable host cell may be generated which is derived from 293 cells (which contain E1 helper functions under the control of a constitutive promoter), but which contain the rep and/or cap proteins under the control of inducible promoters. Still other stable host cells may be generated by one of skill in the art.

In some aspects, the instant disclosure relates to a host cell containing a nucleic acid that comprises a coding sequence encoding a protein (*e*.*g*., a sFasL protein or fragment thereof). In some aspects, the instant disclosure relates to a composition comprising the host cell described above. In some aspects, the composition comprising the host cell above further comprises a cryopreservative.

The recombinant AAV vector, rep sequences, cap sequences, and helper functions required for producing the rAAV of the disclosure may be delivered to the packaging host cell using any appropriate genetic element (vector). The selected genetic element may be delivered by any suitable method, including those described herein. The methods used to construct any aspect of this disclosure are known to those with skill in nucleic acid manipulation and include genetic engineering, recombinant engineering, and synthetic techniques. See, *e.g.*, Sambrook et al., Molecular Cloning: A Laboratory Manual, Cold Spring Harbor Press, Cold Spring Harbor, N.Y. Similarly, methods of generating rAAV virions are well known and the selection of a suitable method is not a limitation on the present disclosure. See, *e.g.,* K. Fisher et al., J. Virol., 70:520-532 (1993) and U.S. Pat. No. 5,478,745.

In some aspects, recombinant AAVs may be produced using the triple transfection method (described in detail in U.S. Pat. No. 6,001,650). Typically, the recombinant AAVs are produced by transfecting a host cell with an recombinant AAV vector (comprising a transgene) to be packaged into AAV particles, an AAV helper function vector, and an accessory function vector. An AAV helper function vector encodes the "AAV helper function" sequences (*i.e.*, rep and cap), which function in *trans* for productive AAV replication and encapsidation. Preferably, the AAV helper function vector supports efficient AAV vector production without generating any detectable wild-type AAV virions (*i.e.*, AAV virions containing functional rep and cap genes). Non-limiting examples of vectors suitable for use with the present disclosure include pHLP19, described in U.S. Pat. No. 6,001,650 and pRep6cap6 vector, described in U.S. Pat. No. 6,156,303. The accessory function vector encodes nucleotide sequences for non-AAV derived viral and/or cellular functions upon which AAV is dependent for replication (*i.e.*, "accessory functions"). The accessory functions include those functions required for AAV replication, including, without limitation, those moieties involved in activation of AAV gene transcription, stage specific AAV mRNA splicing, AAV DNA replication, synthesis of cap expression products, and AAV capsid assembly. Viral-based accessory functions can be derived from any of the known helper viruses such as adenovirus, herpesvirus (other than herpes simplex virus type-1), and vaccinia virus.

In some aspects, the disclosure provides transfected host cells. The term "transfection" is used to refer to the uptake of foreign DNA by a cell, and a cell has been "transfected" when exogenous DNA has been introduced inside the cell membrane. A number of transfection techniques are generally known in the art. See, *e.g.,* Graham et al. (1973) Virology, 52:456, Sambrook et al. (1989) Molecular Cloning, a laboratory manual, Cold Spring Harbor Laboratories, New York, Davis et al. (1986) Basic Methods in Molecular Biology, Elsevier, and Chu et al. (1981) Gene 13:197. Such techniques can be used to introduce one or more exogenous nucleic acids, such as a nucleotide integration vector and other nucleic acid molecules, into suitable host cells.

A "host cell" refers to any cell that harbors, or is capable of harboring, a substance of interest. Often a host cell is a mammalian cell. A host cell may be used as a recipient of an AAV helper construct, an AAV minigene plasmid, an accessory function vector, or other transfer DNA associated with the production of recombinant AAVs. The term includes the progeny of the original cell which has been transfected. Thus, a "host cell" as used herein may refer to a cell which has been transfected with an exogenous DNA sequence. It is understood that the progeny of a single parental cell may not necessarily be completely identical in morphology or in genomic or total DNA complement as the original parent, due to natural, accidental, or deliberate mutation.

As used herein, the term "cell line" refers to a population of cells capable of continuous or prolonged growth and division *in vitro.* Often, cell lines are clonal populations derived from a single progenitor cell. It is further known in the art that spontaneous or induced changes can occur in karyotype during storage or transfer of such clonal populations. Therefore, cells derived from the cell line referred to may not be precisely identical to the ancestral cells or cultures, and the cell line referred to includes such variants.

As used herein, the terms "recombinant cell" refers to a cell into which an exogenous DNA segment, such as DNA segment that leads to the transcription of a biologically-active polypeptide or production of a biologically active nucleic acid such as an RNA, has been introduced.

As used herein, the term "vector" includes any genetic element, such as a plasmid, phage, transposon, cosmid, chromosome, artificial chromosome, virus, virion, *etc.*, which is capable of replication when associated with the proper control elements and which can transfer gene sequences between cells. Thus, the term includes cloning and expression vehicles, as well as viral vectors. In some aspects, useful vectors are contemplated to be those vectors in which the nucleic acid segment to be transcribed is positioned under the transcriptional control of a promoter. A "promoter" refers to a DNA sequence recognized by the synthetic machinery of the cell, or introduced synthetic machinery, required to initiate the specific transcription of a gene. The phrases "operatively positioned," "under control" or "under transcriptional control" means that the promoter is in the correct location and orientation in relation to the nucleic acid to control RNA polymerase initiation and expression of the gene. The term "expression vector or construct" means any type of genetic construct containing a nucleic acid in which part or all of the nucleic acid encoding sequence is capable of being transcribed. In some aspects, expression includes transcription of the nucleic acid, for example, to generate a biologically-active polypeptide product or functional RNA (*e.g.*, guide RNA) from a transcribed gene. The foregoing methods for packaging recombinant vectors in desired AAV capsids to produce the rAAVs of the disclosure are not meant to be limiting and other suitable methods will be apparent to the skilled artisan.

### rAAV-mediated delivery of sFasL transgenes to the eye

Methods for delivering a transgene to ocular (*e*.*g*., photoreceptors, such as rod cells or cone cells, retinal cells, *etc*.) tissue in a subject are provided herein. The methods typically involve administering to a subject an effective amount of a rAAV comprising a nucleic acid for expressing a transgene (*e.g.,* a sFasL protein or fragment thereof) in the subject. An "effective amount" of a rAAV is an amount sufficient to infect a sufficient number of cells of a target tissue in a subject. In some aspects, a target tissue is ocular (*e.g.,* photoreceptor, retinal, *etc.*) tissue. An effective amount of a rAAV may be an amount sufficient to have a therapeutic benefit in a subject, *e.g.*, to improve in the subject one or more symptoms of disease, *e.g.*, a symptom of glaucoma. Examples of a symptom of glaucoma includes, but is not limited to, blind spots in peripheral and/or central vision, tunnel vision, severe headache, eye pain, nausea and vomiting, blurred vision, halos around lights, and eye redness. The effective amount will depend on a variety of factors such as, for example, the species, age, weight, health of the subject, and the ocular tissue to be targeted, and may thus vary among subject and tissue.

An effective amount may also depend on the rAAV used. The disclosure is based, in part on the recognition that certain rAAVs comprising capsid proteins mediate efficient transduction of ocular (*e.g.*, photoreceptor, retinal, *etc.*) cells. In some aspects of the disclosure, an rAAV used in methods provided herein comprises a capsid protein of an AAV serotype selected from the group consisting of: AAV2, AAV5, AAV6, AAV6.2, AAV7, AAV8, AAV9, AAVrh.8, AAVrh.10, AAVrh.39, and AAVrh.43. In some aspects, the rAAV comprises a capsid protein of AAV2 serotype (SEQ ID NO: 1). In some aspects, the capsid protein comprises an amino acid sequence that is at least 70%, at least 80%, at least 90%, at least 95% , or at least 99% identical to SEQ ID NO: 1. In some aspects, the capsid protein is AAV2 capsid protein.

In certain aspects, the effective amount of rAAV is 10¹⁰, 10¹¹, 10¹², 10¹³, or 10¹⁴ genome copies per kg. In certain aspects, the effective amount of rAAV is 10¹⁰, 10¹¹, 10¹², 10¹³, 10¹⁴, or 10¹⁵ genome copies per subject.

An effective amount may also depend on the mode of administration. For example, targeting an ocular (*e.g.,* photoreceptor, retinal, *etc.*) tissue by intrastromal administration or subcutaneous injection may require different (*e.g.*, higher or lower) doses, in some cases, than targeting an ocular (*e.g.*, photoreceptor, retinal, *etc.*) tissue by another method (*e.g.*, systemic administration, topical administration). In some aspects, intrastromal injection (IS) of rAAV having certain serotypes (*e*.*g*., AAV5, AAV6, AAV6.2, AAV7, AAV8, AAV9, AAVrh.8, AAVrh.10, AAVrh.39, and AAVrh.43) mediates efficient transduction of ocular (*e.g.*, corneal, photoreceptor, retinal, *etc.*) cells. Thus, in some aspects, the injection is intrastromal injection (IS). In some aspects, the administration is via injection, optionally subretinal injection or intravitreal injection. In some aspects, the injection is topical administration (*e*.*g*., topical administration to an eye). In some cases, multiple doses of a rAAV are administered.

In some aspects, efficient transduction of ocular (*e.g.*, photoreceptor, retinal, *etc.*) cells by rAAV described herein may be useful for the treatment of a subject having glaucoma (*e.g.*, open-angle glaucoma). Accordingly, methods and compositions for treating glaucoma are also provided herein. In some aspects, the disclosure provides a method for treating glaucoma (*e*.*g*., open-angle glaucoma), the method comprising: administering to a subject having or suspected of having glaucoma an effective amount of rAAV, wherein the rAAV comprises (i) a capsid protein having a serotype selected from the group consisting of AAV2, AAV5, AAV6, AAV6.2, AAV7, AAV8, AAV9, AAVrh.8, AAVrh.10, AAVrh.39, and AAVrh.43, and (ii) a nucleic acid comprising a promoter operably linked to a transgene (*e*.*g*., a transgene encoding a sFasL protein or fragment thereof as described by the disclosure).

In some aspects, administration of a rAAV (or isolated nucleic acid) as described by the disclosure results in transduction of a retinal neuron. Examples of retinal neurons include, but are not limited to, bipolar cells, ganglion cells, horizontal cells, retina amacrine cells, rod cells and cone cells.

In some aspects, administration of a rAAV (or isolated nucleic acid) as described by the disclosure results in transduction of a retinal ganglion cell (RGC). Examples of RGCs include, but are not limited to, W-ganglion cells, X-ganglion cells, Y-ganglion cells, midget cells, parasol cells, bistratified cells, photsensitive ganglion cells, and other ganglion cells projecting to the superior colliculus for eye movements.

Retinal ganglion cells vary significantly in terms of their size, connections, and responses to visual stimulation but they all share the defining property of having a long axon that extends into the brain. These axons form the optic nerve, optic chiasm, and optic tract. In some aspects, administration of a rAAV (or isolated nucleic acid) as described by the disclosure prevents death of a RGC, an axon, or a combination thereof.

The rAAVs may be delivered to a subject in compositions according to any appropriate methods known in the art. The rAAV, preferably suspended in a physiologically compatible carrier (*i.e.*, in a composition), may be administered to a subject, *i.e.* host animal, such as a human, mouse, rat, cat, dog, sheep, rabbit, horse, cow, goat, pig, guinea pig, hamster, chicken, turkey, or a non-human primate (*e*.*g*., Macaque). In some aspects, a host animal does not include a human.

Delivery of the rAAVs to a mammalian subject may be by, for example, intraocular injection or topical administration (*e*.*g*., eye drops). In some aspects, the intraocular injection is intrastromal injection, subconjunctival injection, or intravitreal injection. In some aspects, the injection is not topical administration. Combinations of administration methods (*e*.*g*., topical administration and intrastromal injection) can also be used.

The compositions of the disclosure may comprise an rAAV alone, or in combination with one or more other viruses (*e*.*g*., a second rAAV encoding having one or more different transgenes). In some aspects, a composition comprises 1, 2, 3, 4, 5, 6, 7, 8, 9, 10, or more different rAAVs each having one or more different transgenes.

In some aspects, a composition further comprises a pharmaceutically acceptable carrier. Suitable carriers may be readily selected by one of skill in the art in view of the indication for which the rAAV is directed. For example, one suitable carrier includes saline, which may be formulated with a variety of buffering solutions (*e*.*g*., phosphate buffered saline). Other exemplary carriers include sterile saline, lactose, sucrose, calcium phosphate, gelatin, dextran, agar, pectin, peanut oil, sesame oil, and water. The selection of the carrier is not a limitation of the present disclosure.

Optionally, the compositions of the disclosure may contain, in addition to the rAAV and carrier(s), other pharmaceutical ingredients, such as preservatives, or chemical stabilizers. Suitable exemplary preservatives include chlorobutanol, potassium sorbate, sorbic acid, sulfur dioxide, propyl gallate, the parabens, ethyl vanillin, glycerin, phenol, and parachlorophenol. Suitable chemical stabilizers include gelatin and albumin.

The rAAVs are administered in sufficient amounts to transfect the cells of a desired tissue (*e.g.*, ocular tissue, such as photoreceptor, retinal, *etc.*, tissue) and to provide sufficient levels of gene transfer and expression without undue adverse effects. Examples of pharmaceutically acceptable routes of administration include, but are not limited to, direct delivery to the selected organ (*e*.*g*., subretinal delivery to the eye), oral, inhalation (including intranasal and intratracheal delivery), intraocular, intravenous, intramuscular, subcutaneous, intradermal, intratumoral, and other parental routes of administration. Routes of administration may be combined, if desired.

The dose of rAAV virions required to achieve a particular "therapeutic effect," *e.g.*, the units of dose in genome copies/per kilogram of body weight (GC/kg), will vary based on several factors including, but not limited to: the route of rAAV virion administration, the level of gene or RNA expression required to achieve a therapeutic effect, the specific disease or disorder being treated, and the stability of the gene or RNA product. One of skill in the art can readily determine a rAAV virion dose range to treat a patient having a particular disease or disorder based on the aforementioned factors, as well as other factors.

An effective amount of an rAAV is an amount sufficient to target infect an animal, target a desired tissue. The effective amount will depend primarily on factors such as the species, age, weight, health of the subject, and the tissue to be targeted, and may thus vary among animal and tissue. For example, an effective amount of the rAAV is generally in the range of from about 1 ml to about 100 ml of solution containing from about 10⁹ to 10¹⁶ genome copies. In some cases, a dosage between about 10¹¹ to 10¹³ rAAV genome copies is appropriate. In certain aspects, 10⁹ rAAV genome copies is effective to target ocular tissue (*e.g.*, corneal tissue). In some aspects, a dose more concentrated than 10⁹ rAAV genome copies is toxic when administered to the eye of a subject. In some aspects, an effective amount is produced by multiple doses of an rAAV.

In some aspects, a dose of rAAV is administered to a subject no more than once per calendar day (*e.g.*, a 24-hour period). In some aspects, a dose of rAAV is administered to a subject no more than once per 2, 3, 4, 5, 6, or 7 calendar days. In some aspects, a dose of rAAV is administered to a subject no more than once per calendar week (*e.g.*, 7 calendar days). In some aspects, a dose of rAAV is administered to a subject no more than bi-weekly (*e.g.*, once in a two calendar week period). In some aspects, a dose of rAAV is administered to a subject no more than once per calendar month (*e.g.*, once in 30 calendar days). In some aspects, a dose of rAAV is administered to a subject no more than once per six calendar months. In some aspects, a dose of rAAV is administered to a subject no more than once per calendar year (*e.g.*, 365 days or 366 days in a leap year). In some aspects, a dose of rAAV is administered to a subject no more than once per two calendar years (*e.g.*, 730 days or 731 days in a leap year). In some aspects, a dose of rAAV is administered to a subject no more than once per three calendar years (*e.g.*, 1095 days or 1096 days in a leap year).

In some aspects, rAAV compositions are formulated to reduce aggregation of AAV particles in the composition, particularly where high rAAV concentrations are present (*e.g.*, ~10¹³ GC/ml or more). Appropriate methods for reducing aggregation of may be used, including, for example, addition of surfactants, pH adjustment, salt concentration adjustment, *etc.* (See, *e.g.*, Wright FR, et al., Molecular Therapy (2005) 12, 171-178.)

Formulation of pharmaceutically-acceptable excipients and carrier solutions is well-known to those of skill in the art, as is the development of suitable dosing and treatment regimens for using the particular compositions described herein in a variety of treatment regimens. Typically, these formulations may contain at least about 0.1% of the active compound or more, although the percentage of the active ingredient(s) may, of course, be varied and may conveniently be between about 1 or 2% and about 70% or 80% or more of the weight or volume of the total formulation. Naturally, the amount of active compound in each therapeutically-useful composition may be prepared is such a way that a suitable dosage will be obtained in any given unit dose of the compound. Factors such as solubility, bioavailability, biological half-life, route of administration, product shelf life, as well as other pharmacological considerations will be contemplated by one skilled in the art of preparing such pharmaceutical formulations, and as such, a variety of dosages and treatment regimens may be desirable.

In some aspects, rAAVs in suitably formulated pharmaceutical compositions disclosed herein are delivered directly to target tissue, *e.g.*, direct to ocular tissue (*e.g.*, photoreceptor, retinal, *etc.*,̅ tissue). However, in certain circumstances it may be desirable to separately or in addition deliver the rAAV-based therapeutic constructs via another route, *e*.*g*., subcutaneously, intrapancreatically, intranasally, parenterally, intravenously, intramuscularly, intrathecally, or orally, intraperitoneally, or by inhalation. In some aspects, the administration modalities as described in U.S. Pat. Nos. 5,543,158; 5,641,515 and 5,399,363 may be used to deliver rAAVs. In some aspects, a preferred mode of administration is by intravitreal injection or subretinal injection.

The pharmaceutical forms suitable for injectable use include sterile aqueous solutions or dispersions and sterile powders for the extemporaneous preparation of sterile injectable solutions or dispersions. Dispersions may also be prepared in glycerol, liquid polyethylene glycols, and mixtures thereof and in oils. Under ordinary conditions of storage and use, these preparations contain a preservative to prevent the growth of microorganisms. In many cases the form is sterile and fluid to the extent that easy syringability exists. It must be stable under the conditions of manufacture and storage and must be preserved against the contaminating action of microorganisms, such as bacteria and fungi. The carrier can be a solvent or dispersion medium containing, for example, water, ethanol, polyol (*e*.*g*., glycerol, propylene glycol, and liquid polyethylene glycol, and the like), suitable mixtures thereof, and/or vegetable oils. Proper fluidity may be maintained, for example, by the use of a coating, such as lecithin, by the maintenance of the required particle size in the case of dispersion and by the use of surfactants. The prevention of the action of microorganisms can be brought about by various antibacterial and antifungal agents, for example, parabens, chlorobutanol, phenol, sorbic acid, thimerosal, and the like. In many cases, it will be preferable to include isotonic agents, for example, sugars or sodium chloride. Prolonged absorption of the injectable compositions can be brought about by the use in the compositions of agents delaying absorption, for example, aluminum monostearate and gelatin.

For administration of an injectable aqueous solution, for example, the solution may be suitably buffered, if necessary, and the liquid diluent first rendered isotonic with sufficient saline or glucose. These particular aqueous solutions are especially suitable for intravenous, intramuscular, subcutaneous and intraperitoneal administration. In this connection, a suitable sterile aqueous medium may be employed. For example, one dosage may be dissolved in 1 ml of isotonic NaCl solution and either added to 1000 ml of hypodermoclysis fluid or injected at the proposed site of infusion, (see for example, "Remington's Pharmaceutical Sciences" 15th Edition, pages 1035-1038 and 1570-1580). Some variation in dosage will necessarily occur depending on the condition of the host. The person responsible for administration will, in any event, determine the appropriate dose for the individual host.

Sterile injectable solutions are prepared by incorporating the active rAAV in the required amount in the appropriate solvent with various of the other ingredients enumerated herein, as required, followed by filtered sterilization. Generally, dispersions are prepared by incorporating the various sterilized active ingredients into a sterile vehicle which contains the basic dispersion medium and the required other ingredients from those enumerated above. In the case of sterile powders for the preparation of sterile injectable solutions, the preferred methods of preparation are vacuum-drying and freeze-drying techniques which yield a powder of the active ingredient plus any additional desired ingredient from a previously sterile-filtered solution thereof.

The rAAV compositions disclosed herein may also be formulated in a neutral or salt form. Pharmaceutically-acceptable salts, include the acid addition salts (formed with the free amino groups of the protein) and which are formed with inorganic acids such as, for example, hydrochloric or phosphoric acids, or such organic acids as acetic, oxalic, tartaric, mandelic, and the like. Salts formed with the free carboxyl groups can also be derived from inorganic bases such as, for example, sodium, potassium, ammonium, calcium, or ferric hydroxides, and such organic bases as isopropylamine, trimethylamine, histidine, procaine and the like. Upon formulation, solutions will be administered in a manner compatible with the dosage formulation and in such amount as is therapeutically effective. The formulations are easily administered in a variety of dosage forms such as injectable solutions, drug-release capsules, and the like.

As used herein, "carrier" includes any and all solvents, dispersion media, vehicles, coatings, diluents, antibacterial and antifungal agents, isotonic and absorption delaying agents, buffers, carrier solutions, suspensions, colloids, and the like. The use of such media and agents for pharmaceutical active substances is well known in the art. Supplementary active ingredients can also be incorporated into the compositions. The phrase "pharmaceutically-acceptable" refers to molecular entities and compositions that do not produce an allergic or similar untoward reaction when administered to a host.

Delivery vehicles such as liposomes, nanocapsules, microparticles, microspheres, lipid particles, vesicles, and the like, may be used for the introduction of the compositions of the present disclosure into suitable host cells. In particular, the rAAV vector delivered transgenes may be formulated for delivery either encapsulated in a lipid particle, a liposome, a vesicle, a nanosphere, or a nanoparticle or the like.

Such formulations may be preferred for the introduction of pharmaceutically acceptable formulations of the nucleic acids or the rAAV constructs disclosed herein. The formation and use of liposomes is generally known to those of skill in the art. Recently, liposomes were developed with improved serum stability and circulation half-times (U.S. Pat. No. 5,741,516). Further, various methods of liposome and liposome like preparations as potential drug carriers have been described (U.S. Pat. Nos. 5,567,434; 5,552,157; 5,565,213; 5,738,868 and 5,795,587).

Liposomes have been used successfully with a number of cell types that are normally resistant to transfection by other procedures. In addition, liposomes are free of the DNA length constraints that are typical of viral-based delivery systems. Liposomes have been used effectively to introduce genes, drugs, radiotherapeutic agents, viruses, transcription factors and allosteric effectors into a variety of cultured cell lines and animals. In addition, several successful clinical trials examining the effectiveness of liposome-mediated drug delivery have been completed.

Liposomes are formed from phospholipids that are dispersed in an aqueous medium and spontaneously form multilamellar concentric bilayer vesicles (also termed multilamellar vesicles (MLVs). MLVs generally have diameters of from 25 nm to 4 µm. Sonication of MLVs results in the formation of small unilamellar vesicles (SUVs) with diameters in the range of 200 to 500 Å, containing an aqueous solution in the core.

Alternatively, nanocapsule formulations of the rAAV may be used. Nanocapsules can generally entrap substances in a stable and reproducible way. To avoid side effects due to intracellular polymeric overloading, such ultrafine particles (sized around 0.1 µm) should be designed using polymers able to be degraded *in vivo.* Biodegradable polyalkyl-cyanoacrylate nanoparticles that meet these requirements are contemplated for use.

### Kits and Related Compositions

The agents described herein may, in some embodiments, be assembled into pharmaceutical or diagnostic or research kits to facilitate their use in therapeutic, diagnostic or research applications. A kit may include one or more containers housing the components of the disclosure and instructions for use. Specifically, such kits may include one or more agents described herein, along with instructions describing the intended application and the proper use of these agents. In certain aspects agents in a kit may be in a pharmaceutical formulation and dosage suitable for a particular application and for a method of administration of the agents. Kits for research purposes may contain the components in appropriate concentrations or quantities for running various experiments.

In some aspects, the instant disclosure relates to a kit for producing a rAAV, the kit comprising a container housing an isolated nucleic acid comprising a transgene encoding a sFasL protein or fragment thereof having the amino acid sequence set forth in SEQ ID NO: 3. In some aspects, the kit further comprises a container housing an isolated nucleic acid encoding an AAV capsid protein, for example an AAV2 capsid protein (*e*.*g*., SEQ ID NO: 1).

The kit may be designed to facilitate use of the methods described herein by researchers and can take many forms. Each of the compositions of the kit, where applicable, may be provided in liquid form (*e.g.*, in solution), or in solid form, (*e.g.*, a dry powder). In certain cases, some of the compositions may be constitutable or otherwise processable (*e*.*g*., to an active form), for example, by the addition of a suitable solvent or other species (for example, water or a cell culture medium), which may or may not be provided with the kit. As used herein, "instructions" can define a component of instruction and/or promotion, and typically involve written instructions on or associated with packaging of the disclosure. Instructions also can include any oral or electronic instructions provided in any manner such that a user will clearly recognize that the instructions are to be associated with the kit, for example, audiovisual (*e*.*g*., videotape, DVD, *etc.*), Internet, and/or web-based communications, *etc.* The written instructions may be in a form prescribed by a governmental agency regulating the manufacture, use or sale of pharmaceuticals or biological products, which instructions can also reflects approval by the agency of manufacture, use or sale for animal administration.

The kit may contain any one or more of the components described herein in one or more containers. As an example, in one aspect, the kit may include instructions for mixing one or more components of the kit and/or isolating and mixing a sample and applying to a subject. The kit may include a container housing agents described herein. The agents may be in the form of a liquid, gel or solid (powder). The agents may be prepared sterilely, packaged in syringe and shipped refrigerated. Alternatively it may be housed in a vial or other container for storage. A second container may have other agents prepared sterilely. Alternatively the kit may include the active agents premixed and shipped in a syringe, vial, tube, or other container.

Exemplary aspects of the disclosure will be described in more detail by the following examples. These aspects are exemplary of the disclosure, which one skilled in the art will recognize is not limited to the exemplary aspects.

### EXAMPLES

### Example 1: High levels of mFasL coincide with activation of Fas-mediated apoptotic and non-apoptotic inflammatory pathways

### MATERIALS AND METHODS

### Animals

All animal experiments were approved by the Institutional Animal Care and Use Committee at Schepens Eye Research Institute and were performed under the guidelines of the Association of Research in Vision and Ophthalmology (Rockville, MD). The DBA/2J-Gpnmb⁺/SjJ mice (D2-Gp) were purchased. DBA/2J mice expressing the ΔCS mutation were produced by crossing the ΔCS founder mice to DBA/2J mice purchased from Jackson Laboratories for 10 generations. After 10 generations, D2.ΔCS mice heterozygous for the ΔCS mutation were intercrossed to produce D2.ΔCS homozygous for the ΔCS mutation (D2.ΔCS) mice and WT littermates (D2). All three genotypes (D2, D2. ΔCS, and D2.Gp) were housed and maintained under cyclic light (12L-30 lux: 12D) condition in an AAALAC approved animal facility at the Schepens Eye Research Institute.

### IOP measurements

IOP was measured with a rebound TonoLab tonometer (Colonial Medical Supply, Espoo, Finland). Mice were anesthetized by 3% isoflurane in 100% oxygen (induction) followed by 1.5% isoflurane in 100% oxygen (maintenance) delivered with a precision vaporizer. IOP measurement was initiated within 2 to 3 min after animals lost toe pinch reflex or tail pinch response. Anesthetized mice were placed on a platform and the tip of the pressure sensor was placed approximately 1/8 inch from the central cornea. Average IOP was displayed automatically after 6 measurements after elimination of the highest and lowest values. This machine-generated mean was considered as one reading, and six readings were obtained for each eye. For D2.Gp, D2, and D2.ΔCS mice, IOPs were measured once a month. For the microbead study, baseline IOPs were obtained one day before microbead injection and the IOPs were then measured every three days after microbead injection. All IOPs were taken at the same time of day (between 10:00 and 12:00 hours) due to the variation of IOP throughout the day.

### Quantification of retinal ganglion cells

The neural retina was isolated and fixed in 4% paraformaldehyde for 2 hour at room temperature. Retinal flat mounts were incubated with a primary antibody against a RGC-specific marker and β-III-tubulin (Millipore,Billerica, MA) at 4 °C overnight. An Alexa Fluor 594-conjugated secondary antibody (Invitrogen) was used as secondary antibody. Nuclei were counterstained with DAPI. A 60X oil-immersion was used and sixteen non-overlapping images were taken, with four-five images within each quadrant. All cells in ganglion cell layer positively labeled by the β-III-tubulin antibody were counted. Retinal areas were measured using Image J software, and the RGC density/mm² retina was calculated.

### Quantification of optic nerve axons

For quantification of axons, optic nerves were dissected and fixed in Karnovsky's reagent (50% in phosphate buffer) over night. Semithin cross-sections of the nerve were taken at 1.0 mm posterior to the globe were dissected and fixed in Karnovsky solution (50% in phosphate buffer) overnight. Ultrathin (60-90 nm) optic nerve cross-sections were prepared and stained with 1% p-phenylenediamine (PPD) for evaluation by light microscopy. Ten non-overlapping photomicrographs were taken at 100X magnification covering the entire area of the optic nerve cross-section. The total area of each optic nerve cross-section was determined with Image J software and this value was used to estimate the total axon density per optic nerve cross-section.

### TUNEL staining

Apoptotic cells were evaluated by TUNEL staining. Eyes were enucleated, fixed in 10% formalin. Eyes were processed and 20 µm paraffin sections mounted on slides were used for staining. The sections were de-paraffinized before staining for TUNEL according to the manufacturer's protocol (In Situ Cell Death Detection Kit; Roche Applied Science, Mannheim Germany, 11684795910). The sections were mounted using VECTARSHIELD Mounting Medium with DAPI (Vector Laboratories, H-1200). Sections were washed, cover slipped and examined by confocal laser scanning microscopy (Leica Microsystems and processed using SP6 software).

### Quantitative RT-PCR

At the time point for euthanasia anesthetized mice were perfused through the left ventricle with 10 ml of 1xPBS. Following perfusion, the eyes were enucleated and RNA was isolated from the neural retina using Qiagen RNeasy mini kit (Cat # 74104) according to the manufacturer's protocol. RNA was treated with DNAse (Cat # AM222, Invitrogen) to ensure no contamination of genomic DNA. 500ng of RNA was used to prepare cDNA using the iScript cDNA synthesis kit from Bio-Rad (Cat # 170-8890) according to the manufacturer's protocol. cDNA was treated with RNaseH (18021-014, Invitrogen) to ensure absence of single stranded RNA. Quantitative real time PCR was performed using the master mix from Invitrogen (Cat # 4472903) using the manufacturer's protocol in 10µl total volume in duplicates. Relative expression to housekeeping gene β-actin was quantified using the formula: Relative expression =10,000 × 1/2^(Avg. Gene cT- Avg. β-actin cT). All of the primers used are listed in Table 1.

**Table 1. List of RNA Primers Used for Real-time PCR.**

| | Primers | Sequence | SEQ ID NO: |
|---|---|---|---|
| Forward | TNF-α | 5'-GGG ACA GTG ACC TGG ACT GT-3' | 8 |
| Reverse | TNF-α | 5'-CTC CCT TTG CAG AAC TCA GG-3' | 9 |
| Forward | β-actin | 5'-TGT TAC CAA CTG GGA CGA CA-3' | 10 |
| Reverse | β-actin | 5'-CTT TTC ACG GTT GGC CTT AG-3' | 11 |
| Forward | C-FLIP | 5'-TTC TGA TAT AGG GTC CTG C-3' | 12 |
| Reverse | C-FLIP | 5'-TCA CCA GAT CCA AGA AAC TC-3' | 13 |
| Forward | FADD | 5'-CAA GCT GAG TGT AAC TGA AG-3' | 14 |
| Reverse | FADD | 5'-TTA AAA GGC ATC AGC AAG AG-3' | 15 |
| Forward | GFAP | 5'-GGC GCT CAA TGC TGG CTT CA-3' | 16 |
| Reverse | GFAP | 5'-TCT GCC TCC AGC CTC AGG TT-3' | 17 |
| Forward | BAX | 5'- AGG GTT TCA TCC AGG ATC GAG CAG-3' | 18 |
| Reverse | BAX | 5'-ATC TTC CAG ATG GTG AGC GAG-3' | 19 |
| Forward | BCL-2 | 5'TTG TGG CCT TCT TTG AGT TCG GTG-3' | 20 |
| Reverse | BCL-2 | 5' GGT GCC GGT TCA GGT ACT CAG TCA-3' | 21 |
| Forward | TRADD | GAA GTT CCC GGT TTC CTC TC | 22 |
| Reverse | TRADD | GAG GGC AGG ATC TCT CAG TG | 23 |
| Forward | c-IAP-2 | TGT CAG CCA AGT TCA AGC TG | 24 |
| Reverse | c-IAP-2 | ATC TTC CGA ACT TTC TCC AGG G | 25 |
| Forward | Fas-L | TGG GTA GAC AGC AGT GCC AC | 26 |
| Reverse | Fas-L | GCC CAC AAG ATG GAC AGG G | 27 |
| Forward | Fas- R | GTC CTG CCT CTG GTG CTT GCT G | 28 |
| Reverse | Fas-R | CAG GTT GGC ATG GTT GA | 29 |

### Western blot analysis

To assess total FasL expression in the posterior segments of D2, D2.Gp, and D2.ΔCS mice, protein lysates (20µg/sample) were prepared from posterior eye cups (neural retina, choroid, and sclera). To assess over expression of sFasL in the neural retina of AAV2 treated eyes, protein lysates (5ug/sample) were prepared from the neural retina only. Proteins were separated on 12% Tris-glycine gels (Invitrogen, Carlsbad CA) and transferred to polyvinylidene difluoride membranes (Invitrogen, Carlsbad CA). The membranes were probed for Fas ligand using a polyclonal rabbit anti-Fas ligand antibody followed by an IRDye secondary antibody (Li-Cor Biotechnology, Lincoln NE). Mouse β actin was used to assess equal loading. Each blot was developed using the LI-COR Odyssey imaging system (LI-COR Biotechnology, Lincoln NE). Densitometry was performed using Image Studio software. L5178Y-R tumor transfectants over expressing sFasL or mFasL were used as positive controls and a protein lysate prepared from a posterior eyecup prepared from FasL-KO mice was used as a negative control.

### Statistics

All data are presented as mean ± SEM. Graph pad prism 6 (La Jolla, CA, USA). For comparisons two-way ANOVA, one-way ANOVA and Tukey's multiple-comparison test was used for D2-AAV2.eGFP and D2-AAV2.sFasL and Sidak's multiple-comparison test for microbead-induced model of elevated IOP. A P value less than 0.05 were considered significant.

### RESULTS

### Accelerated glaucoma in D2.ΔCS mice

DBA/2J (D2) mice spontaneously develop age-related elevated intraocular pressure (IOP) due to mutations in Gpnmb and Tyrp1 genes that trigger iris stromal atrophy and pigment dispersion, respectively. As a result, D2 mice develop elevated IOP by approximately 6-8 months of age, followed by the loss of RGCs and nerve fibers. It was demonstrated that DBA/2J mice expressing only membrane form of FasL (D2.ΔCS) displayed marked thinning of the nerve fiber layer at 5 months of age that was not seen in D2 mice that expressed WT FasL. Early thinning of the nerve fiber layer suggested that the development of glaucoma was accelerated.

To demonstrate the accelerated development of glaucoma in the D2.ΔCS mice, disease progression in young and old D2.ΔCS mice was compared with D2 littermates that express WT FasL (positive control) and DBA/2J congenic mice that express a normal Gpnmb gene (D2-Gp) and develop a very mild form of iris disease due to the Tyrp1 mutation, but do not develop elevated intraocular pressure (IOP) or glaucoma (negative control). IOP was measured using rebound tonometry at 3, 6 and 9 months of age (FIG. 1A). D2 and D2.ΔCS mice displayed elevated IOP beginning at 6 months of age, as compared with the negative control D2.Gp mice. However, there were no significant differences in IOP between D2 and D2.ΔCS mice at any age, indicating the ΔCS mutation does not affect IOP.

To determine whether the loss of RGCs and/or axons was accelerated in D2.ΔCS mice, groups of mice were euthanized at 3 and 6 months of age and retinal flat mounts were stained with the RGC-specific marker βIII tubulin to assess RGC density and optic nerve sections were stained with paraphenylenediamine (PPD) to assess axon density. The loss of RGCs and axons in D2 mice is age-related and only develops in mice >8 months old. Therefore, at 3 and 6 months of age D2 displayed no significant loss of RGCs (FIGs. 1B-1C) or axons (FIGs. 1D-1E), as compared with D2.Gp mice. By contrast, D2.ΔCS mice developed early loss of RGCs (FIGs. 1B-1C) and axons (FIGs. 1D-1E) at 6 months, indicating expression of non-cleavable FasL in D2.ΔCS mice results in accelerated development of glaucoma.

To demonstrate the accelerated death of RGCs in D2.ΔCS mice was due to apoptosis, TUNEL staining was performed on retinal sections from D2, D2.ΔCS, and D2.Gp mice at 3 and 6 months of age. TUNEL+ cells were detected in the RGC layer of D2.ΔCS at both 3 months of age (FIGs. 2A-2B) and 6 months of age (FIGs. 2C-2D), as compared with D2 and D2.Gp mice. Moreover, at 6 months of age, the TUNEL positive cells detected in D2.ΔCS mice were no longer restricted to the RGC layer (FIG. 2C). Together, these results indicate that D2.ΔCS mice display early death of RGCs via apoptosis and loss of axons, resulting in accelerated development of glaucoma.

### Expression of Fas, FasL and FADD in D2.ΔCS mice

To determine the effects of elevated IOP on components of the Fas-induced apoptosis pathway in D2 and D2.ΔCS mice, the retina was analyzed by quantitative RT-PCR for the mRNA levels of Fas, FasL, and FADD and Western blot was used to determine the relative expression of mFasL and sFasL.

There was no significant difference in Fas mRNA expression at either 3 or 6 months of age in D2, D2.ΔCS, or D2.Gp mice (FIG. 3A). By contrast, 6 month old D2.ΔCS mice displayed a significant increase in mRNA for FasL and FADD, a mediator of the extrinsic pathway of Fas-induced apoptosis (FIGs. 3B-3C). Because mFasL expression was detected while sFasL expression was undetectable in D2.ΔCS mice at 6 months of age (FIGs. 3D-3F), the increase in FasL was due to increased expression of mFasL. Undetectable expression of FasL is consistent with the knock-in mutation of the FasL cleavage site in D2.ΔCS mice preventing expression of sFasL within the retina. In addition, this experiment revealed in mice that possessed the WT allele for FasL (D2.Gp and D2 mice) that sFasL was the predominant form of FasL expressed in the retina, indicating that in the eye, under homeostatic conditions, most FasL is cleaved to the soluble form.

Taken together, these data demonstrate the accelerated loss of RGCs observed in D2-ΔCS mice at 6 months coincides with an increased expression of mFasL and induction of the mediators of the extrinsic pathway of Fas-induced apoptosis.

### Muller cell activation in D2-ΔCS mice

Muller cells, which span all retinal layers, constitute the principal glial cells of the retina and provide support to retinal neurons. In the normal retina, glial fibrillary acidic protein (GFAP) is expressed in the nerve fiber layer by astrocytes and the end feet of Muller cells. However, in glaucoma, GFAP expression is significantly increased in the muller cells as a result of reactive gliosis and is considered an indicator of neuroinflammation.

To determine whether accelerated development of glaucoma in D2.ΔCS mice coincided with early activation of pro-inflammatory Muller cells, GFAP expression in the retina was measured at 3 and 6 months of age in D2, D2.ΔCS, and D2.Gp mice by RT-PCR and immunohistochemical staining using an anti-GFAP antibody. At 3 months of age, GFAP expression was restricted to the astrocytes and Muller cell end feet in the nerve fiber layer in all three groups of mice and there no significant difference in expression of GFAP between D2, D2.ΔCS, and D2.Gp mice (FIGs. 4A-4B). However, by 6 months of age, expression of GFAP in D2.ΔCS mice was no longer limited to the astrocytes and muller end feet in the nerve fiber layer, but extended throughout the whole length of retinal Muller cells and this coincided with a significant increase in GFAP mRNA levels, as compared to D2 and D2.Gp mice (FIGs. 4A-4B). The increase in GFAP coincided with a significant increase in the TNFα mRNA levels in D2.ΔCS mice as compared to D2 and D2.Gp mice (FIG. 4C). TNFα is produced by activated glial cells in glaucoma and is linked to glial activation, inflammation, and mediation of RGC death. These data indicate that accelerated loss of RGCs in D2.ΔCS mice that express high levels of mFasL coincides, not only with the induction of apoptosis of RGCs, but also with glial activation and the induction of TNFα. Therefore, high levels of mFasL in D2.ΔCS mice coincides with activation of Fas-mediated apoptotic and non-apoptotic inflammatory pathways.

### Example 2: Delivery of sFasL provides therapeutic effects in a mouse model of glaucoma

### MATERIALS AND METHODS

### Viral vector construction

The Adeno-associated vectors, scAAV2-CB6-PI-eGFP and scAAV2-CB6-PI-sFasLecto, were prepared. The scAAV2-CB6-PI-sFasLecto vector has a nucleic acid sequence as set forth in SEQ ID NO: 5. The seed plasmid pAAVsc CB6 PI sFasLecto was made by isolating a 582 bp fragment corresponding to the full cDNA sequence of sFasL ecto 5'GCSF from pcDNA3-sFasL-ecto plus 5' mGCSF. The 582 bp fragment was ligated (T4 ligase NEB #M0202) to a BamHI, HindIII double digested and Antartic phosphatase treated pAAVsc-CB6-PI plasmid. An aliquot of the ligation mixture was transfected onto *E.coli* competent cells and plated onto LB-Agar plus 50 ug/ml carbenicillin. Colonies were picked and grown on LB-carbenicillin media, and plasmid mini-preps were checked by restriction enzyme and sequencing. A large scale of the plasmid was prepared from 1 liter of bacterial culture and isolated using a ZR Plamid gigaprep kit (#D4056, Zymo Research, Irvine CA).

### Intravitreal injections

The intravitreal injections, just posterior to the limbus-parallel conjunctival vessels, were performed. Mice received a 1µl intravitreal injection into both eyes containing AAV2CB6.sFasL (3×10⁹ PFU/ml) or AAV2CB6.eGFP (3×10⁹ PFU/ml) and sterile physiologic saline was used as a vehicle control.

### RESULTS

### Intravitreal delivery of sFasL using AAV2

As shown herein, the membrane-bound form of FasL is responsible for both the induction of apoptosis and for proinflammatory cytokine production. Therefore, the exacerbated development of glaucoma in the D2.ΔCS mice is easily explained by the increased expression of uncleaved mFasL. However, sFasL has been shown to serve as an mFasL antagonist and to actually block FasL-induced inflammation.

To determine whether sFasL could also block the effects of mFasL in glaucoma, an adeno-associated virus serotype 2 (AAV2) vector was used to deliver sFasL to the RGCs of D2 mice via a single intravitreal injection. It has been demonstrated that intravitreal administration of AAV2 vectors in mice primarily results in the transduction of long-lived RGCs and cells of the inner nuclear layer. As a result, gene delivery using AAV2 can provide long-term persistent expression.

To evaluate which retinal cells were transduced by AAV2, an AAV2 vector expressing only eGFP (AAV.eGFP) was injected intravitreally into two month-old D2 mice. Confocal microscopy was used to assess eGFP expression in retinal whole mounts at 2 weeks post injection and showed uniform eGFP distribution throughout the retina (FIG. 5A). Cross sectional reconstruction of the retina showed strong eGFP expression throughout the ganglion cell layer and inner nuclear layers (FIG. 5B). D2 mice received similar injections of AAV2.sFasL and Western blot analysis of sFasL in the neural retina showed a significant increase in sFasL expression as early as 2 weeks post injection, as compared to D2 mice treated with either AAV2.eGFP or uninjected control mice (FIG. 5C). Moreover, the increase in sFasL in the retina was sustained at a consistent level throughout the length of the study (8 months) following a single injection. Monthly monitoring of IOP revealed no significant differences in IOP levels at any age between D2 uninjected control mice and D2 mice that received either AAV2.eGFP or AAV2.sFasL treatment (FIG. 5D). Furthermore, representative slit lamp images taken at 9 months of age showed no differences in the severity of either iris pigment dispersion or iris atrophy between D2 uninjected control mice and D2 mice that received either AAV2.eGFP or AAV2.sFasL treatment (FIG. 5D).

Taken together, these results demonstrate that a single intravitreal injection of AAV2.sFasL into D2 mice produces long-term stable expression of high levels of sFasL within the inner retina, which has no effect on the development of iris pigment dispersion, iris atrophy, and elevated IOP.

### Intravitreal delivery of AAV2.sFasL prevents loss of RGCs and axons in D2 mice

To determine whether over expression of sFasL prevents the loss of RGCs and axons in D2 mice, mice received an intravitreal injection of AAV2.sFasL at 2 months of age and were euthanized 8 months later at 10 months of age. RGC density was measured in retinal whole mounts stained with βIII tubulin and axon density was measured in optic nerve sections. There was a significant decrease in both RGC and axon density in D2-uninjected mice and D2 AAV2.eGFP mice that develop glaucoma when compared to the D2.Gp negative control mice (FIGs. 6A-6B). By contrast, D2 mice treated with AAV2.sFasL displayed no significant loss in either RGC or axon density as compared with D2.Gp control mice that don't develop glaucoma (FIG. 6A). These data demonstrate that over expression of sFasL in the retina prevented development of glaucoma and loss of RGCs and axons in D2 mice, even in the presence of elevated IOP.

### sFasL prevents activation of pro-inflammatory Muller cells in D2 mice

As shown herein, loss of RGCs in glaucoma coincides with activation of pro-inflammatory Muller cells that express GFAP and the induction of TNFα. To determine whether the absence of glaucoma in AAV2.sFasL treated D2 mice coincided with reduced Muller cell activation, GFAP expression in Muller cells was determined by immunohistochemical staining and RT-PCR was used to measure GFAP and TNFα mRNA expression in the neural retina of 10 month old D2-untreated, D2.AAV2.eGFP, D2.AAV2.sFasL, and D2.Gp mice.

As shown herein, D2-untreated and D2.AAV2.eGFP mice that develop glaucoma displayed activated pro-inflammatory Muller cells that express high levels of GFAP as seen in confocal images (FIG. 4D) and RT-PCR revealed a significant increase in GFAP and TNFα mRNA expression (FIGs. 4E-4F). By contrast, in D2.AAV2.sFasL mice that do not develop glaucoma Muller cells express significantly less GFAP and TNFα mRNA levels were equivalent to those seen in D2.Gp normal control mice (FIGs. 4D-4F). These data indicate that in D2.AAV2.sFasL mice the sFasL-mediated protection of RGCs and axons coincides with preventing activation of pro-inflammatory Muller cells.

### sFasL prevents upregulation of apoptotic genes and downregulation of pro-survival genes in D2 mice

In glaucoma, both extrinsic and intrinsic pathways of apoptosis have been identified as mediators of RGC apoptosis. To determine whether treatment with sFasL inhibited the activation of both extrinsic and intrinsic pathways of apoptosis, qRT-PCR was performed on retinal tissue from D2, D2.Gp, D2-AAV2.eGFP, and D2-AAV2.sFasL mice at 10 months of age to assess the expression of apoptotic genes and pro-survival genes in both the extrinsic and intrinsic pathways. At 10 months of age, the pro-apoptotic mediators Fas, FADD, and BAX were elevated in D2 and D2-AAV2.eGFP mice (FIG. 7A). However, treatment with AAV2.sFasL prevented the upregulation of these genes and the mRNA levels of Fas, FADD, and BAX in D2-AAV2.sFasL mice were equal to the levels detected in the D2.Gp control mice (FIG. 7A). Similarly, the levels of the pro-survival genes cFLIP, BCL2, and cIAP-2 (FIG. 7B) were down regulated in D2 and D2 AAV2.eGFP mice, and treatment with AAV2.sFasL prevented the downregulation of these genes and the mRNA levels of cFLIP, Bcl2, and cIAP2 in D2-AAV2.sFasL mice were equal to the levels detected in the D2.Gp control mice. These results demonstrate that the presence of sFasL blocks FasL-mediated downregulation of pro-survival genes and FasL mediated up-regulation apoptotic genes, even in the presence of elevated IOP.

### Example 3: sFasL treatment provides therapeutic effects when administered after evidence of RGC damage

Data provided herein indicates that treatment of D2 mice with AAV2.sFasL provided significant protection to the RGCs and their axons when compared to untreated and AAV2.eGFP control groups (FIGs. 6A-6B). However, these mice were only followed out to 10 months of age.

To demonstrate that AAV2.sFasL treatment provides long-term protection and does not just delay the death of RGCs and axons, groups of mice were followed for up to 15 months of age. Quantification of RGCs and axons revealed that even at 15 months of age, the AAV2.sFasL treated D2 mice continued to display significant protection of RGCs and axons when compared D2-uninjected mice and AAV2.eGFP mice (FIGs. 8A-8B). These data demonstrate that treatment of pre-glaucomatous D2 mice with AAV2.sFasL results in complete and sustained protection of RGCs and axons, even in the presence of elevated IOP.

However, the more clinically relevant question is whether AAV2.sFasL treatment can provide significant protection when given after RGC damage is detected. Evidence of RGC apoptosis as early as 6 months of age in D2 mice has been reported, and this is in agreement with the TUNEL staining reported herein (FIGs. 2A-2D). Therefore, 7 month old D2 mice were treated with AAV2.sFasL or AAV2.eGFP as a negative control and the mice were followed out to 15 months of age. Quantification of the RGCs and axons at 15 months of age revealed significant preservation of both RGCs and axons when compared to untreated and AAV2.eGFP control groups (FIGs. 8C-8D).

These data demonstrate that overexpression of sFasL in the neural retina provides significant and long-term protection to both the RGCs and axons of D2 mice, even when administered after evidence of RGC damage.

### Example 4: sFasL treatment provides therapeutic effects in a microbead-induced model of glaucoma

### MATERIALS AND METHODS

### Induction of elevated IOP

Mice were anesthetized by intraperitoneal injection of a mixture of ketamine (100 mg/kg; Ketaset; Fort Dodge Animal Health, Fort Dodge, IA) and xylazine (9 mg/kg; TranquiVed; Vedco, Inc., St. Joseph, MO) supplemented by topical application of proparacaine (0.5%; Bausch & Lomb, Tampa, FL). Elevation of IOP was induced unilaterally by injection of polystyrene microbeads (FluoSpheres; Invitrogen, Carlsbad, CA; 15-µm diameter) to the anterior chamber of the right eye of each animal under a surgical microscope. Briefly, microbeads were prepared at a concentration of 5.0 × 10⁶ beads/mL in sterile physiologic saline. The right cornea was gently punctured near the center using a sharp glass micropipette (World Precision Instruments Inc., Sarasota, FL). A small volume (2 µL) of microbeads was injected through this preformed hole into the anterior chamber followed by injection of an air bubble via the micropipette connected with a Hamilton syringe. Any mice that developed signs of inflammation (clouding of the cornea, edematous cornea etc.) were excluded from the study.

### RESULTS

To determine if the neuroprotective effect of sFasL was unique to the DBA/2J model of glaucoma, the ability of sFasL to prevent RGC loss in a microbead-induced mouse model of elevated IOP was investigated. C57BL/6 mice received an intravitreal injection of AAV2.eGFP or AAV2.sFasL followed by anterior chamber injection of microbeads or saline as a negative control.

At three weeks post AAV2 injection, Western blot analysis confirmed overexpression of sFasL in the neural retina of the AAV2.sFasL mice as compared to the AAV2.eGFP mice (FIG. 9A). A single anterior chamber injection of 15µm polystyrene microbeads resulted in elevated IOP for up to 21 days in both AAV2.eGFP and AAV2.sFasL mice as compared to saline controls (FIG. 9B). IOPs peaked at 11 days post microbead injection and there was no significant difference in the time course or magnitude of the microbead-induced elevated IOP between AAV2.sFasL or AAV2.eGFP treated mice. Quantification of RGC density at 4 weeks post microbead injection revealed a significant decrease in RGC density in microbead-injected AAV2.eGFP treated mice as compared to the saline-injected control (FIGs. 9C-9D). However, treatment with AAV2.sFasL protected RGCs and the RGC density in microbead-injected AAV2.sFasL mice was equal to the RGC density in the saline-injected controls (FIGs. 9C-9D). Similar results were observed in the optic nerve where AAV2.sFasL treatment afforded complete protection of the axons as compared to the AAV2.eGFP treated control group (FIGs. 9E-9F).

These results demonstrate that AAV2.sFasL provides complete neuroprotection in both the chronic DBA/2J and the acute microbead-induced models of elevated IOP.

### SEQUENCES

**SEQ ID NO: 1 -** AAV type 2 Capsid Protein Sequence
**SEQ ID NO: 2** - Human Soluble Fas Ligand (Accession No.: NM_000639.2; Nucleic Acid Residues 576-1040) DNA Sequence 
GAGAAGCAAATAGGCCACCCCAGTCCACCCCCTGAAAAAAAGGAGCTGAGGAAAGTGGCCCA
TTTAACAGGCAAGTCCAACTCAAGGTCCATGCCTCTGGAATGGGAAGACACCTATGGAATTG
TCCTGCTTTCTGGAGTGAAGTATAAGAAGGGTGGCCTTGTGATCAATGAAACTGGGCTGTAC
TTTGTATATTCCAAAGTATACTTCCGGGGTCAATCTTGCAACAACCTGCCCCTGAGCCACAA
GGTCTACATGAGGAACTCTAAGTATCCCCAGGATCTGGTGATGATGGAGGGGAAGATGATGA
GCTACTGCACTACTGGGCAGATGTGGGCCCGCAGCAGCTACCTGGGGGCAGTGTTCAATCTT
ACCAGTGCTGATCATTTATATGTCAACGTATCTGAGCTCTCTCTGGTCAATTTTGAGGAATC
TCAGACGTTTTTCGGCTTATATAAGCTCTAA
**SEQ ID NO: 3** - Human Soluble Fas Ligand (Accession No.: P48023.1; Amino Acids 128-281) Protein Sequence 
EKQIGHPSPPPEKKELRKVAHLTGKSNSRSMPLEWEDTYGIVLLSGVKYKKGGLVINETGLY
FVYSKVYFRGQSCNNLPLSHKVYMRNSKYPQDLVMMEGKMMSYCTTGQMWARSSYLGAVFNL
TSADHLYVNVSELSLVNFEESQTFFGLYKL
**SEQ ID NO: 4** - Simian Virus 40 (SV40) Promoter DNA Sequence 
TCTCCCCCCCCTCCCCACCCCCAATTTTGTATTTATTTATTTTTTAATTATTTTGTGCAGCG
ATGGGGGCGGGGGGGGGGGGGGGGGGGGCGCGCGCCAGGCGGGGCGGGGCGGGGCGAGGGGC
GGGGCGGGGCGAGGCGGAGAGGTGCGGCGGCAGCCAATCAGAGCGGCGCGCTCCGAAAGTTT
CCTTTTATGGCGAGGCGGCGGCGGCGGCGGCCCTATAAAAAGCGAAGCGCGCGGCGGGCGGG
AGCGGGATCAGCCACCGCGGTGGCGGCCCTAGAGTCGATCGAGGAACTGAAAAACCAGAAAG
TTAACTGGTAAGTTTAGTCTTTTTGTCTTTTATTTCAGGTCCCGGATCC
**SEQ ID NO: 5** - pAAVsc-CB6-PI-sFasLecto Vector Sequence 
CTGCGCGCTCGCTCGCTCACTGAGGCCGCCCGGGCAAAGCCCGGGCGTCGGGCGACCTTTGG
TCGCCCGGCCTCAGTGAGCGAGCGAGCGCGCAGAGAGGGACGCGCGAGCGAGCGAGTGACTC
CGGCGGGCCCGTTTCGGGCCCGCAGCCCGCTGGAAACCAGCGGGCCGGAGTCACTCGCTCGC
TCGCGCGTCTCTCCGAGTGTAGCCATGCTCTAGGAAGATCAATTCGGTACAATTCACGCGTC
GACATTGATTATTGACTCTGGTCGTTACATAACTTACGGTAAATGGCCCGCCCTCACATCGG
TACGAGATCCTTCTAGTTAAGCCATGTTAAGTGCGCAGCTGTAACTAATAACTGAGACCAGC
AATGTATTGAATGCCATTTACCGGGCGGTGGCTGACCGCCCAACGACCCCGCCCATTGACGT
CAATAATGACGTATGTTCCCATAGTAACGCCAATAGGGACTTTCCATTGACGTCAATGGGTG
GAGTACCGACTGGCGGGTTGCTGGGGCGGGTAACTGCAGTTATTACTGCATACAAGGGTATC
ATTGCGGTTATCCCTGAAAGGTAACTGCAGTTACCCACCTATTTACGGTAAACTGCCCACTT
GGCAGTACATCAAGTGTATCATATGCCAAGTACGCCCCCTATTGACGTCAATGACGGTAAAT
GGCCCGCCTGGCATTATAAATGCCATTTGACGGGTGAACCGTCATGTAGTTCACATAGTATA
CGGTTCATGCGGGGGATAACTGCAGTTACTGCCATTTACCGGGCGGACCGTAATTGCCCAGT
ACATGACCTTATGGGACTTTCCTACTTGGCAGTACATCTACTCGAGGCCACGTTCTGCTTCA
CTCTCCCCATCTCCCCCCCCTCCCCACCCCACGGGTCATGTACTGGAATACCCTGAAAGGAT
GAACCGTCATGTAGATGAGCTCCGGTGCAAGACGAAGTGAGAGGGGTAGAGGGGGGGGAGGG
GTGGGGCAATTTTGTATTTATTTATTTTTTAATTATTTTGTGCAGCGATGGGGGCGGGGGGGG
GGGGGGGGGGGGCGCGCGCCAGGCGGGGCGGGGCGGGGCGAGGGTTAAAACATAAATAAAT
AAAAAATTAATAAAACACGTCGCTACCCCCGCCCCCCCCCCCCCCCCCCCCGCGCGCGGTCC
GCCCCGCCCCGCCCCGCTCCGGCGGGGCGGGGCGAGGCGGAGAGGTGCGGCGGCAGCCAATC
AGAGCGGCGCGCTCCGAAAGTTTCCTTTTATGGCGAGGCGGCGGCGGCGGCGGCCCTACCGC
CCCGCCCCGCTCCGCCTCTCCACGCCGCCGTCGGTTAGTCTCGCCGCGCGAGGCTTTCAAAG
GAAAATACCGCTCCGCCGCCGCCGCCGCCGGGATTAAAAAGCGAAGCGCGCGGCGGGCGGGA
GCGGGATCAGCCACCGCGGTGGCGGCCCTAGAGTCGATCGAGGAACTGAAAAACCAGAAAGT
TAACTGGTAAATTTTTCGCTTCGCGCGCCGCCCGCCCTCGCCCTAGTCGGTGGCGCCACCGC
CGGGATCTCAGCTAGCTCCTTGACTTTTTGGTCTTTCAATTGACCATTGTTTAGTCTTTTTG
TCTTTTATTTCAGGTCCCCGGATCCATGGCTCAACTTTCTGCCCAGAGGCGCATGAAGCTAA
TGGCCCTGCAGCTGCTGCTGTGGCCAAATCAGAAAAACAGAAAATAAAGTCCAGGGGCCTAG
GTACCGAGTTGAAAGACGGGTCTCCGCGTACTTCGATTACCGGGACGTCGACGACGACACCG
AAAGTGCACTATGGTCAGGACGAGAGGCCGTTCCCCTGGTCACTGTCAGCGCTCTGGAATTC
GAAAAGCAAATAGCCAACCCCAGTACACCCTCTGAAAATTTCACGTGATACCAGTCCTGCTC
TCCGGCAAGGGGACCAGTGACAGTCGCGAGACCTTAAGCTTTTCGTTTATCGGTTGGGGTCA
TGTGGGAGACTTTTAAAAGAGCCGAGGAGTGTGGCCCATTTAACAGGGAACCCCCACTCAAG
GTCCATCCCTCTGGAATGGGAAGACACATATGGAACCGCTCTGATCTCTGGATTTTCTCGGC
TCCTCACACCGGGTAAATTGTCCCTTGGGGGTGAGTTCCAGGTAGGGAGACCTTACCCTTCT
GTGTATACCTTGGCGAGACTAGAGACCTGTGAAGTATAAGAAAGGTGGCCTTGTGATCAACG
AAACTGGGTTGTACTTCGTGTATTCCAAAGTATACTTCCGGGGTCAGTCCTGCAACAACCAG
CCCCCACTTCATATTCTTTCCACCGGAACACTAGTTGCTTTGACCCAACATGAAGCACATAA
GGTTTCATATGAAGGCCCCAGTCAGGACGTTGTTGGTCGGGGTAAACCACAAGGTCTATATG
AGGAACTCTAAGTATCCTGAGGATCTGGTGCTAATGGAGGAGAAGAGGTTGAACTACTGCAC
TACTGGACAGATATGGGCATTTGGTGTTCCAGATATACTCCTTGAGATTCATAGGACTCCTA
GACCACGATTACCTCCTCTTCTCCAACTTGATGACGTGATGACCTGTCTATACCCGCCACAG
CAGCTACCTGGGGGCAGTATTCAATCTTACCAGTGCTGACCATTTATATGTCAACATATCTC
AACTCTCTCTGATCAATTTTGAGGAATCTAAGGGTGTCGTCGATGGACCCCCGTCATAAGTT
AGAATGGTCACGACTGGTAAATATACAGTTGTATAGAGTTGAGAGAGACTAGTTAAAACTCC
TTAGATTCACCTTTTTCGGCTTATATAAGCTTATCGATACCGTCGACTAGAGCTCGCTGATC
AGCCTCGACTGTGCCTTCTAGTTGCCAGCCATCTGTTGTTTGCCCCTGGAAAAAGCCGAATA
 
TATTCGAATAGCTATGGCAGCTGATCTCGAGCGACTAGTCGGAGCTGACACGGAAGATCAAC
GGTCGGTAGACAACAAACGGGGTCCCCCGTGCCTTCCTTGACCCTGGAAGGTGCCACTCCCA
CTGTCCTTTCCTAATAAAATGAGGAAATTGCATCGCATTGTCTGAGTAGGTGTCATTCTAAG
GGGGCACGGAAGGAACTGGGACCTTCCACGGTGAGGGTGACAGGAAAGGATTATTTTACTCC
TTTAACGTAGCGTAACAGACTCATCCACAGTAAGATTTCTGGGGGGTGGGGTGGGGCAGGAC
AGCAAGGGGGAGGATTGGGAAGACAATTAGGTAGATAAGTAGCATGGCGGGTTAATCATTAA
CTACAAGGAACCAAGACCCCCCACCCCACCCCGTCCTGTCGTTCCCCCTCCTAACCCTTCTG
TTAATCCATCTATTCATCGTACCGCCCAATTAGTAATTGATGTTCCTTGGCCTAGTGATGGA
GTTGGCCACTCCCTCTCTGCGCGCTCGCTCGCTCACTGAGGCCGGGCGACCAAAGGTCGCCC
GACGCCCGGGCTTTGCCCGGGCGGCCGGATCACTACCTCAACCGGTGAGGGAGAGACGCGCG
AGCGAGCGAGTGACTCCGGCCCGCTGGTTTCCAGCGGGCTGCGGGCCCGAAACGGGCCCGCC
GGTCAGTGAGCGAGCGAGCGCGCAGCCTTAATTAACCTAATTCACTGGCCGTCGTTTTACAA
CGTCGTGACTGGGAAAACCCTGGCGTTACCCAACTTAATCAGTCACTCGCTCGCTCGCGCGT
CGGAATTAATTGGATTAAGTGACCGGCAGCAAAATGTTGCAGCACTGACCCTTTTGGGACCG
CAATGGGTTGAATTAGGCCTTGCAGCACATCCCCCTTTCGCCAGCTGGCGTAATAGCGAAGA
GGCCCGCACCGATCGCCCTTCCCAACAGTTGCGCAGCCTGAATGGCGAATGGGACGGAACGT
CGTGTAGGGGGAAAGCGGTCGACCGCATTATCGCTTCTCCGGGCGTGGCTAGCGGGAAGGGT
TGTCAACGCGTCGGACTTACCGCTTACCCTCGCGCCCTGTAGCGGCGCATTAAGCGCGGCGG
GTGTGGTGGTTACGCGCAGCGTGACCGCTACACTTGCCAGCGCCCTAGCGCCCGCTCCTTTC
GCTTTCGCGCGGGACATCGCCGCGTAATTCGCGCCGCCCACACCACCAATGCGCGTCGCACT
GGCGATGTGAACGGTCGCGGGATCGCGGGCGAGGAAAGCGAAAGTTCCCTTCCTTTCTCGCC
ACGTTCGCCGGCTTTCCCCGTCAAGCTCTAAATCGGGGGCTCCCTTTAGGGTTCCGATTTAG
TGCTTTACGGCACCTCGACCAAGGGAAGGAAAGAGCGGTGCAAGCGGCCGAAAGGGGCAGTT
CGAGATTTAGCCCCCGAGGGAAATCCCAAGGCTAAATCACGAAATGCCGTGGAGCTGGCCAA
AAAACTTGATTAGGGTGATGGTTCACGTAGTGGGCCATCGCCCTGATAGACGGTTTTTCGCC
CTTTGACGTTGGAGTCCACGTTCTTTAATAGTGGGGTTTTTTGAACTAATCCCACTACCAAG
TGCATCACCCGGTAGCGGGACTATCTGCCAAAAAGCGGGAAACTGCAACCTCAGGTGCAAGA
AATTATCACCACTCTTGTTCCAAACTGGAACAACACTCAACCCTATCTCGGTCTATTCTTTT
GATTTATAAGGGATTTTGCCGATTTCGGCCTATTGGTTAAAAAATGAGTGAGAACAAGGTTT
GACCTTGTTGTGAGTTGGGATAGAGCCAGATAAGAAAACTAAATATTCCCTAAAACGGCTAA
AGCCGGATAACCAATTTTTTACTCCTGATTTAACAAAAATTTAACGCGAATTTTAACAAAAT
ATTAACGCTTACAATTTAGGTGGCACTTTTCGGGGAAATGTGCGCGGAACCCCTATTTGTTT
GACTAAATTGTTTTTAAATTGCGCTTAAAATTGTTTTATAATTGCGAATGTTAAATCCACCG
TGAAAAGCCCCTTTACACGCGCCTTGGGGATAAACAAAATTTTTCTAAATACATTCAAATAT
GTATCCGCTCATGAGACAATAACCCTGATAAATGCTTCAATAATATTGAAAAAGGAAGAGTA
TGAGTATTCAACATTAAAAAGATTTATGTAAGTTTATACATAGGCGAGTACTCTGTTATTGG
GACTATTTACGAAGTTATTATAACTTTTTCCTTCTCATACTCATAAGTTGTATTCCGTGTCG
CCCTTATTCCCTTTTTTGCGGCATTTTGCCTTCCTGTTTTTGCTCACCCAGAAACGCTGGTG
AAAGTAAAAGATGCTGAAGATCAGTTGGAAGGCACAGCGGGAATAAGGGAAAAAACGCCGTA
AAACGGAAGGACAAAAACGAGTGGGTCTTTGCGACCACTTTCATTTTCTACGACTTCTAGTC
AACCGTGCACGAGTGGGTTACATCGAACTGGATCTCAACAGCGGTAAGATCCTTGAGAGTTT
TCGCCCCGAAGAACGTTTTCCAATGATGAGCACTTTTAAAGTCACGTGCTCACCCAATGTAG
CTTGACCTAGAGTTGTCGCCATTCTAGGAACTCTCAAAAGCGGGGCTTCTTGCAAAAGGTTA
CTACTCGTGAAAATTTCATCTGCTATGTGGCGCGGTATTATCCCGTATTGACGCCGGGCAAG
AGCAACTCGGTCGCCGCATACACTATTCTCAGAATGACTTGGTTGAGTACTCACCAAGACGA
TACACCGCGCCATAATAGGGCATAACTGCGGCCCGTTCTCGTTGAGCCAGCGGCGTATGTGA
TAAGAGTCTTACTGAACCAACTCATGAGTGGTGTCACAGAAAAGCATCTTACGGATGGCATG
 
ACAGTAAGAGAATTATGCAGTGCTGCCATAACCATGAGTGATAACACTGCGGCCAACTTACT
TCTGACAACAGTGTCTTTTCGTAGAATGCCTACCGTACTGTCATTCTCTTAATACGTCACGA
CGGTATTGGTACTCACTATTGTGACGCCGGTTGAATGAAGACTGTTCGATCGGAGGACCGAA
GGAGCTAACCGCTTTTTTGCACAACATGGGGGATCATGTAACTCGCCTTGATCGTTGGGAAC
CGGAGCTGAATGAAGCCATACCGCTAGCCTCCTGGCTTCCTCGATTGGCGAAAAAACGTGTT
GTACCCCCTAGTACATTGAGCGGAACTAGCAACCCTTGGCCTCGACTTACTTCGGTATGGAA
ACGACGAGCGTGACACCACGATGCCTGTAGCAATGGCAACAACGTTGCGCAAACTATTAACT
GGCGAACTACTTACTCTAGCTTCCCGGCAACAATTATTTGCTGCTCGCACTGTGGTGCTACG
GACATCGTTACCGTTGTTGCAACGCGTTTGATAATTGACCGCTTGATGAATGAGATCGAAGG
GCCGTTGTTAATATAGACTGGATGGAGGCGGATAAAGTTGCAGGACCACTTCTGCGCTCGGC
CCTTCCGGCTGGCTGGTTTATTGCTGATAAATCTGGAGCCGGTGAGCGTGTATCTGACCTAC
CTCCGCCTATTTCAACGTCCTGGTGAAGACGCGAGCCGGGAAGGCCGACCGACCAAATAACG
ACTATTTAGACCTCGGCCACTCGCACGGTCTCGCGGTATCATTGCAGCACTGGGGCCAGATG
GTAAGCCCTCCCGTATCGTAGTTATCTACACGACGGGGAGTCAGGCAACTATGGATGAACGA
AACCAGAGCGCCATAGTAACGTCGTGACCCCGGTCTACCATTCGGGAGGGCATAGCATCAAT
AGATGTGCTGCCCCTCAGTCCGTTGATACCTACTTGCTTTTAGACAGATCGCTGAGATAGGT
GCCTCACTGATTAAGCATTGGTAACTGTCAGACCAAGTTTACTCATATATACTTTAGATTGA
TTTAAAACTTCATTTTATCTGTCTAGCGACTCTATCCACGGAGTGACTAATTCGTAACCATT
GACAGTCTGGTTCAAATGAGTATATATGAAATCTAACTAAATTTTGAAGTAAAATAATTTAA
AAGGATCTAGGTGAAGATCCTTTTTGATAATCTCATGACCAAAATCCCTTAACGTGAGTTTT
CGTTCCACTGAGCGTCAGACCCCGTAGAAAATTAAATTTTCCTAGATCCACTTCTAGGAAAA
ACTATTAGAGTACTGGTTTTAGGGAATTGCACTCAAAAGCAAGGTGACTCGCAGTCTGGGGC
ATCTTTAGATCAAAGGATCTTCTTGAGATCCTTTTTTTCTGCGCGTAATCTGCTGCTTGCAA
ACAAAAAAACCACCGCTACCAGCGGTGGTTTGTTTGCCGGATCATCTAGTTTCCTAGAAGAA
CTCTAGGAAAAAAAGACGCGCATTAGACGACGAACGTTTGTTTTTTTGGTGGCGATGGTCGC
CACCAAACAAACGGCCTAGTAGAGCTACCAACTCTTTTTCCGAAGGTAACTGGCTTCAGCAG
AGCGCAGATACCAAATACTGTTCTTCTAGTGTAGCCGTAGTTAGGCCACCACTTCAAGTCTC
GATGGTTGAGAAAAAGGCTTCCATTGACCGAAGTCGTCTCGCGTCTATGGTTTATGACAAGA
AGATCACATCGGCATCAATCCGGTGGTGAAGTTCAACTCTGTAGCACCGCCTACATACCTCG
CTCTGCTAATCCTGTTACCAGTGGCTGCTGCCAGTGGCGATAAGTCGTGTCTTACCGGGTTG
GACTCAAGACTTGAGACATCGTGGCGGATGTATGGAGCGAGACGATTAGGACAATGGTCACC
GACGACGGTCACCGCTATTCAGCACAGAATGGCCCAACCTGAGTTCTGGATAGTTACCGGAT
AAGGCGCAGCGGTCGGGCTGAACGGGGGGTTCGTGCACACAGCCCAGCTTGGAGCGAACGAC
CTACACCGAACTGAGATACCTACACTATCAATGGCCTATTCCGCGTCGCCAGCCCGACTTGC
CCCCCAAGCACGTGTGTCGGGTCGAACCTCGCTTGCTGGATGTGGCTTGACTCTATGGATGT
GCGTGAGCTATGAGAAAGCGCCACGCTTCCCGAAGGGAGAAAGGCGGACAGGTATCCGGTAA
GCGGCAGGGTCGGAACAGGAGAGCGCACGAGGGAGCTTCGCACTCGATACTCTTTCGCGGTG
CGAAGGGCTTCCCTCTTTCCGCCTGTCCATAGGCCATTCGCCGTCCCAGCCTTGTCCTCTCG
CGTGCTCCCTCGAACCAGGGGGAAACGCCTGGTATCTTTATAGTCCTGTCGGGTTTCGCCAC
CTCTGACTTGAGCGTCGATTTTTGTGATGCTCGTCAGGGGGGCGGAGCCTATGGTCCCCCTT
TGCGGACCATAGAAATATCAGGACAGCCCAAAGCGGTGGAGACTGAACTCGCAGCTAAAAAC
ACTACGAGCAGTCCCCCCGCCTCGGATAGGAAAAACGCCAGCAACGCGGCCTTTTTACGGTT
CCTGGCCTTTTGCTGGCCTTTTGCTCACATGTTCTTTCCTGCGTTATCCCCTGATTCTGTGG
ATAACCTTTTTGCGGTCGTTGCGCCGGAAAAATGCCAAGGACCGGAAAACGACCGGAAAACG
AGTGTACAAGAAAGGACGCAATAGGGGACTAAGACACCTATTCCGTATTACCGCCTTTGAGT
GAGCTGATACCGCTCGCCGCAGCCGAACGACCGAGCGCAGCGAGTCAGTGAGCGAGGAAGCG
GAAGAGCGCCCAATACGCGGCATAATGGCGGAAACTCACTCGACTATGGCGAGCGGCGTCGG
 
CTTGCTGGCTCGCGTCGCTCAGTCACTCGCTCCTTCGCCTTCTCGCGGGTTATGCGAAACCG

CCTCTCCCCGCGCGTTGGCCGATTCATTAATGCAGCTGGCACGACAGGTTTCCCGACTGGAA
AGCGGGCAGTGAGCGCAACGCAATTAATGTGATTTGGCGGAGAGGGGCGCGCAACCGGCTAA
GTAATTACGTCGACCGTGCTGTCCAAAGGGCTGACCTTTCGCCCGTCACTCGCGTTGCGTTA
ATTACACTTCATTAGGCACCCCAGGCTTTACACTTTATGCTTCCGGCTCGTATGTTGTGTGG
AATTGTGAGCGGATAACAATTTCACACAGGAAACAGCAATCGAGTGAGTAATCCGTGGGGTC
CGAAATGTGAAATACGAAGGCCGAGCATACAACACACCTTAACACTCGCCTATTGTTAAAGT
GTGTCCTTTGTCCTATGACCATGATTACGCCAGATTTAATTAAGGCCTTAATTAGGGATACT
GGTACTAATGCGGTCTAAATTAATTCCGGAATTAATCC
**SEQ ID NO: 6** - Human Fas Ligand (Accession No.: P48023.1; Amino Acids 1-281) Protein Sequence 
MQQPFNYPYPQIYWVDSSASSPWAPPGTVLPCPTSVPRRPGQRRPPPPPPPPPPLPPPPPPPP
LPPLPLPPLKKRGNHSTGLCLLVMFFMVLVALVGLGLGMFQLFHLQKELAELRESTSQMHTA
SSLEKQIGHPSPPPEKKELRKVAHLTGKSNSRSMPLEWEDTYGIVLLSGVKYKKGGLVINET
GLYFVYSKVYFRGQSCNNLPLSHKVYMRNSKYPQDLVMMEGKMMSYCTTGQMWARSSYLGAV
FNLTSADHLYVNVSELSLVNFEESQTFFGLYKL
**SEQ ID NO: 7** - Human Fas Ligand (Accession No.: NM_000639.2; Nucleic Acid Residues 195-1040) DNA Sequence

ATGCAGCAGCCCTTCAATTACCCATATCCCCAGATCTACTGGGTGGACAGCAGTGCCAGCTC
TCCCTGGGCCCCTCCAGGCACAGTTCTTCCCTGTCCAACCTCTGTGCCCAGAAGGCCTGGTC
AAAGGAGGCCACCACCACCACCGCCACCGCCACCACTACCACCTCCGCCGCCGCCGCCACCA
CTGCCTCCACTACCGCTGCCACCCCTGAAGAAGAGAGGGAACCACAGCACAGGCCTGTGTCT
CCTTGTGATGTTTTTCATGGTTCTGGTTGCCTTGGTAGGATTGGGCCTGGGGATGTTTCAGC
TCTTCCACCTACAGAAGGAGCTGGCAGAACTCCGAGAGTCTACCAGCCAGATGCACACAGCA
TCATCTTTGGAGAAGCAAATAGGCCACCCCAGTCCACCCCCTGAAAAAAAGGAGCTGAGGAA
AGTGGCCCATTTAACAGGCAAGTCCAACTCAAGGTCCATGCCTCTGGAATGGGAAGACACCT
ATGGAATTGTCCTGCTTTCTGGAGTGAAGTATAAGAAGGGTGGCCTTGTGATCAATGAAACT
GGGCTGTACTTTGTATATTCCAAAGTATACTTCCGGGGTCAATCTTGCAACAACCTGCCCCT
GAGCCACAAGGTCTACATGAGGAACTCTAAGTATCCCCAGGATCTGGTGATGATGGAGGGGA
AGATGATGAGCTACTGCACTACTGGGCAGATGTGGGCCCGCAGCAGCTACCTGGGGGCAGTG
TTCAATCTTACCAGTGCTGATCATTTATATGTCAACGTATCTGAGCTCTCTCTGGTCAATTT
TGAGGAATCTCAGACGTTTTTCGGCTTATATAAGCTCTAA

## Claims

1. A recombinant adeno-associated virus (rAAV) for use in a method of treating a Fas ligand-dependent inflammatory condition in the eye of a subject, wherein the rAAV comprises:
(a) an AAV2 capsid protein; and
(b) a nucleic acid engineered to express soluble Fas ligand (sFasL).

2. The rAAV for use according to claim 1, wherein the Fas ligand-dependent inflammatory condition is glaucoma.

3. The rAAV for use according to claim 2, wherein the subject has an elevated intraocular pressure (IOP).

4. The rAAV for use according to any one of claims 2 to 3, wherein the subject is human.

5. The rAAV for use according to any one of claims 2 to 4, wherein the administration results in delivery of the rAAV to the eye of the subject.

6. The rAAV for use according to any one of claims 2 to 5, wherein the rAAV is administered via injection, optionally subretinal injection, or intravitreal injection.

7. The rAAV for use according to any one of claims 2 to 5, wherein the rAAV is administered via topical administration to the eye of a subject.

8. The rAAV for use according to any one of claims 2 to 7, wherein the rAAV is administered to the subject no more than once in 15 months.

9. The rAAV for use according to any one of claims 2 to 8, wherein administration results in reducing glaucoma disease progression.

10. The rAAV for use according to any one of claims 2 to 9, wherein the administration results in lowering intraocular pressure in the subject.

11. The rAAV for use according to any one of claims 2 to 10, wherein the administration results in inactivating retinal glial cells in the subject.

12. The rAAV for use of any one of claims 2 to 11, wherein the administration results in inhibiting TNF-alpha activity in the subject.

13. The rAAV for use according to any one of claims 2 to 12, wherein the administration results in reducing retinal ganglion cell (RGC) death and/or reducing axonal degeneration.

14. The rAAV for use according to claim 2, wherein the eye of the subject has retinal ganglion cell (RGC) death and/or axonal degeneration.

15. The rAAV for use according to claim 2, wherein the subject has one or more of the following symptoms of glaucoma: blind spots in peripheral and/or central vision, tunnel vision, eye pain, blurred vision, halos around lights, or eye redness.

## Patentansprüche

1. Rekombinantes adenoassoziiertes Virus (rAAV) zur Verwendung in einem Verfahren zur Behandlung eines Fas-Ligand-bedingten Entzündungsleidens im Auge eines Individuums, wobei das rAAV Folgendes umfasst:
(a) ein AAV2-Kapsid-Protein; und
(b) eine Nucleinsäure, die so manipuliert wurde, dass sie löslichen Fas-Liganden (sFasL) exprimiert.

2. rAAV zur Verwendung nach Anspruch 1 wobei das Fas-Ligand-bedingte Entzündungsleiden Glaukom ist.

3. rAAV zur Verwendung nach Anspruch 2, wobei das Individuum einen erhöhten Augeninnendruck (IOP) aufweist.

4. rAAV zur Verwendung nach einem der Ansprüche 2 bis 3, wobei das Individuum ein Mensch ist.

5. rAAV zur Verwendung nach einem der Ansprüche 2 bis 4, wobei die Verabreichung zur Abgabe des rAAV an das Auge des Individuums führt.

6. rAAV zur Verwendung nach einem der Ansprüche 2 bis 5, wobei das rAAV mittels Injektion, gegebenenfalls subretinaler Injektion oder intravitrealer Injektion, verabreicht wird.

7. rAAV zur Verwendung nach einem der Ansprüche 2 bis 5, wobei das rAAV mittels topischer Verabreichung an das Auge des Individuums verabreicht wird.

8. rAAV zur Verwendung nach einem der Ansprüche 2 bis 7, wobei das rAAV nicht öfter als einmal alle 15 Monate an das Individuum verabreicht wird.

9. rAAV zur Verwendung nach einem der Ansprüche 2 bis 8, wobei die Verabreichung zur Verringerung des Fortschreitens einer Glaukom-Erkrankung führt.

10. rAAV zur Verwendung nach einem der Ansprüche 2 bis 9, wobei die Verabreichung zur Senkung des Augeninnendrucks des Individuums führt.

11. rAAV zur Verwendung nach einem der Ansprüche 2 bis 10, wobei die Verabreichung zur Inaktivierung der Netzhaut-Gliazellen in dem Individuum führt.

12. rAAV zur Verwendung nach einem der Ansprüche 2 bis 11, wobei die Verabreichung zur Inhibierung der TNF-alpha-Aktivität in dem Individuum führt.

13. rAAV zur Verwendung nach einem der Ansprüche 2 bis 12, wobei die Verabreichung zur Verringerung des Absterbens von Ganglionzellen der Netzhaut (RGC) und/oder zur Verringerung von axonaler Degeneration führt.

14. rAAV zur Verwendung nach Anspruch 2, wobei das Auge des Individuums an einem Absterben der Ganglionzellen der Netzhaut (RGC) und/oder einer axonalen Degeneration leidet.

15. rAAV zur Verwendung nach Anspruch 2, wobei das Individuum eines oder mehrere der folgenden Glaukom-Symptome aufweist: blinde Flecken im peripheren und/oder zentralen Sichtfeld, Tunnelblick, Augenschmerzen, verschwommenes Sehen, Lichthöfe rund um Lichter oder Augenrötung.

## Revendications

1. Virus adéno-associé recombinant (AAVr) à utiliser dans un procédé de traitement d'un état inflammatoire dépendant du ligand Fas dans l'œil d'un sujet, dans lequel l'AAVr comprend :
(a) une protéine de capside AAV2 ; et
(b) un acide nucléique conçu pour exprimer un ligand Fas soluble (sFasL).

2. AAVr à utiliser selon la revendication 1, dans lequel l'état inflammatoire dépendant du ligand Fas est un glaucome.

3. AAVr à utiliser selon la revendication 2, dans lequel le sujet a une pression intraoculaire élevée (TOP).

4. AAVr à utiliser selon l'une quelconque des revendications 2 à 3, dans lequel le sujet est humain.

5. AAVr à utiliser selon l'une quelconque des revendications 2 à 4, dans lequel l'administration engendre une libération du AAVr dans l'oeil du sujet.

6. AAVr à utiliser selon l'une quelconque des revendications 2 à 5, dans lequel le AAVr est administré par injection, facultativement par injection sous-rétinienne, ou par injection intravitréenne.

7. AAVr à utiliser selon l'une quelconque des revendications 2 à 5, dans lequel l'AAVr est administré par administration topique dans l'oeil d'un sujet.

8. AAVr à utiliser selon l'une quelconque des revendications 2 à 7, dans lequel le AAVr est administré au sujet pas plus d'une fois en 15 mois.

9. AAVr à utiliser selon l'une quelconque des revendications 2 à 8, dans lequel l'administration engendre une réduction de la progression de la maladie du glaucome.

10. AAVr à utiliser selon l'une quelconque des revendications 2 à 9, dans lequel l'administration engendre une baisse de pression intraoculaire chez le sujet.

11. AAVr à utiliser selon l'une quelconque des revendications 2 à 10, dans lequel l'administration engendre une inactivation de cellules gliales de la rétine chez le sujet.

12. AAVr à utiliser selon l'une quelconque des revendications 2 à 11, dans lequel l'administration engendre l'inhibition de l'activité TNF-alpha chez le sujet.

13. AAVr à utiliser selon l'une quelconque des revendications 2 à 12, dans lequel l'administration engendre une réduction de la mort des cellules ganglionnaires rétiniennes (CGR) et/ou une réduction de la dégénérescence axonale.

14. AAVr à utiliser selon la revendication 2, dans lequel l'œil du sujet présente une mort des cellules ganglionnaires rétiniennes (CGR) et/ou une dégénérescence axonale.

15. AAVr à utiliser selon la revendication 2, dans lequel le sujet présente un ou plusieurs des symptômes suivants du glaucome : taches aveugles dans la vision périphérique et/ou centrale, vision tunnel, douleur oculaire, vision floue, halos autour des lumières ou rougeur oculaire.
